# EUROPÄISCHE PATENTSCHRIFT

(11) **EP 3 695 797 B1**
(45) Veröffentlichungstag und Bekanntmachung des Hinweises auf die Patenterteilung: **10.01.2024**
(21) Anmeldenummer: 20157564.4
(22) Anmeldetag: 17.02.2020
(51) Int. Cl.: A61B 17/88, B01F 31/40, B01F 33/501

(54) **KNOCHENZEMENTAPPLIKATOR MIT KLEMMBAREM AUSTRAGSKOLBEN**
BONE CEMENT APPLICATOR WITH CLAMPABLE DISCHARGE PLUNGER
APPLICATEUR DE CIMENT OSSEUX POURVU DE PISTON DE DÉCHARGE POUVANT ÊTRE SERRÉ

(30) Priorität: 18.02.2019 DE 102019104020
(43) Veröffentlichungstag der Anmeldung: 19.08.2020
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- EP-A1- 2 974 681
- EP-A1- 3 231 505
- US-A- 5 558 136

## Beschreibung

Die Erfindung betrifft einen Knochenzementapplikator zum Herstellen eines Knochenzements aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einem solchen Knochenzementapplikator.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2, der EP 0 796 653 A2, der US 5 588 745 A, der US 2018/333 176 A1, der US 2018/310 974 A1, der US 2018/289 406 A1, der US 2018/132 919 A1, der US 2018/132 917 A1 und der US 2018/256 233 A1 vorgeschlagen.

Die EP 3 231 505 A1 offenbart ein Kartuschensystem mit einem arretierbaren Austragskolben. Hierzu ist eine lösbare Rasteinrichtung vorgesehen, die durch einen axialen Druck lösbar ist. Zu einem ähnlichen Zweck ist bei der EP 2 974 681 A1 am Innenumfang einer Kartusche eine umlaufende Feder in Form eines Rings vorgesehen, die in zwei Gegenrastmittel in Form von umlaufenden Nuten vorgesehen. In beiden Fällen erfolgt die Rastung durch einen Formschluss eines Rastmittels der Kartusche mit einem Gegenrastmittel am Kolben.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschließen einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig angewendet. Bei Verwendung von Mischvorrichtungen befindet sich der Knochenzementteig im Fall von Pulver-Flüssigkeits-Zementen in einer Kartusche. Bei der Herstellung solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar.

Bei einfachen Vakuummischsystemen muss der Austragskolben während des Mischvorgangs in der Kartusche verriegelt sein, um ein Herausziehen des Austragskolbens während des Mischens durch ein Mischorgan zu verhindern. Weiterhin sollte der Austragskolben in der Kartusche fixiert sein, damit durch ein anliegendes Vakuum der Austragskolben nicht in das Innere der Kartusche gesaugt wird.

Zur Fixierung des Austragskolbens wurden bisher formschlüssige Verriegelungen vorgeschlagen.

Im WO 90/13264 A1 wurde eine Nut-Feder-Verrastung des Austragskolbens vorgeschlagen. Der verrastete Austragskolben wird durch axiale Krafteinwirkung einer Austragsvorrichtung aus seinem Sitz gerissen. Dadurch kommt der Austragskolben frei und kann den gemischten Zementteig aus der Kartusche auspressen. Weitere Nut-Feder-Verbindungen wurden in den Druckschriften EP 0 397 589 B1 und DE 29 21 565 A1 offenbart.

In der Patentanmeldung WO 01/85070 A1 wird eine manuell zu entfernende Lasche beschrieben, die in Stege des Austragskolbens eingreift, um diesen zu verrasten.

Die DE 10 2012 024 710 A1 offenbart ein Mischsystem für einen Knochenzement, bei dem ein Austragskolben mit einem Mischstab verriegelt werden kann, um eine Bewegung des Mischstabs gegen den Austragskolben zu verhindern. Eine Verrastung des Austragskolbens gegen die Kartusche ist mit der Verriegelung des Mischstabs an dem Austragskolben nicht möglich, da der Mischstab, wie auch der Austragskolben gegen die Kartusche beweglich sind. In der DE 10 2007 026 034 A1 wird ein Rastring beschrieben, der an seiner Innenseite Rastelemente und an seiner Außenseite Gegenrastelemente besitzt. Die äußeren Rastelemente greifen in eine Nut der Kartusche und die inneren Rastelemente in eine Nut des Austragskolbens. Bei axialer Krafteinwirkung auf den Austragskolben wird die Verrastung der äußeren oder der inneren Rastelemente überwunden und der Austragskolben kann axial in der Kartusche verschoben werden.

In der WO 2004/100771 A2 wurde ein Austragskolben beschrieben, der Rastelemente besitzt, die bei Druckbeaufschlagung des Austragskolbens durch einen hohlkegelförmigen Teller einer Austragsvorrichtung nach innen aus der Verrastung gezogen werden.

Nachteilig ist an diesen Systemen, dass eine Rastung in einem sehr stark begrenzten Bereich wirkt. Die Systeme weisen daher keine Toleranz hinsichtlich der Verschiebung des Austragskolbens auf. Sobald der Punkt der Rastung überfahren ist, ist der Austragskolben gelöst. Es kann daher zu Fehlbedienungen kommen, bei denen beim Einsetzen des Austragskolbens oder beim Einrasten des Austragskolbens der Punkt der Rastung versehentlich überfahren wird und dadurch beim Mischen des Knochenzements der Austragskolben im nicht gerasteten Zustand beweglich ist.

Ferner sind einige der Rastvorrichtungen aufwendig im Aufbau. Es besteht immer das Bedürfnis nach kostengünstigen aber gleichzeitig zuverlässigen Lösungen.

Die Aufgabe der vorliegenden Erfindung besteht darin, die Nachteile des Stands der Technik zu überwinden. Insbesondere besteht die Aufgabe der Erfindung in der Entwicklung eines Knochenzementapplikators und eines Verfahrens bei dem der Austragskolben auf kostengünstige aber zuverlässige Weise lösbar arretiert werden kann. Das Lösen des Austragskolbens soll vorzugsweise ohne zusätzliche Arbeitsschritte erfolgen. Der Knochenzementapplikator und das Verfahren sollen zum Mischen des Knochenzements aus den Ausgangskomponenten und zum Austragen des gemischten Knochenzements vorgesehen und geeignet sein. Der Aufbau soll kostengünstig sein, damit die Vorrichtung aus hygienischen Gründen nur einmalig verwendet werden kann.

Die Aufgabe der Erfindung besteht auch in der Entwicklung eines Knochenzementapplikators zum Vermischen und Austragen von Polymethylmethacrylat-Knochenzement. Der zu entwickelnde Knochenzementapplikator soll eine Vermischung des Polymethylmethacrylat-Knochenzementpulvers mit der Monomerflüssigkeit im Innenraum einer Kartusche ermöglichen.

Die Vermischung kann dabei durch eine manuell von außen zu betätigende Mischstange mit einem daran angebrachten Mischorgan bewirkt werden. Der Mischstab kann dazu durch den Austragskolben geführt sein. Dann ist es notwendig, den Austragskolben in der Kartusche mechanisch so zu fixieren, dass der Austragskolben beim Mischprozess nicht aus der Kartusche gezogen werden kann. Weiterhin ist es wichtig, wenn beim Vermischen der Zementkomponenten unter angelegtem Vakuum der Austragskolben nicht in den Innenraum der Kartusche infolge der Vakuumeinwirkung gezogen werden kann. Nach dem Mischen soll der Austragskolben vorzugsweise durch Einwirkung einer Auspressvorrichtung gelöst werden können. Dazu soll die Kartusche mit dem Austragskolben so gestaltet werden, dass ohne eine formschlüssige Rastverbindung eine Fixierung des Austragskolbens kraftschlüssig bewirkt wird. Diese kraftschlüssige Fixierung muss dann so gestaltet sein, dass nach dem Herausdrücken des Austragskolbens aus dem Bereich der Fixierung durch Einwirkung der Auspressvorrichtung der Austragskolben ohne Bremswirkung durch Teile der Fixierung entlang der Innenseite der Kartusche gleiten kann.

Weiterhin soll der Austragskolben so fixierbar sein, dass ein Herausdrücken aus dem Bereich der Fixierung manuell ohne Hilfsmittel nicht möglich ist. Ein Herausdrücken soll nur mit einer mechanischen Auspressvorrichtung möglich sein, die üblicherweise zum Auspressen von Knochenzement benutzt wird.

Der Knochenzementapplikator soll dabei so gestaltet sein, dass er als Prepacked-Kartuschensystem und auch als vom medizinischen Anwender zu befüllendes Mischsystem geeignet ist. Im Fall der Verwendung des zu entwickelnden Knochenzementapplikators als Prepacked-Mischsystem soll ein zweiteiliger Austragskolben gemäß der Lehre der DE 10 2009 031 178 B3 verwendet werden können.

Die Aufgaben der Erfindung werden gelöst durch einen Knochenzementapplikator zum Bereitstellen eines Knochenzements, insbesondere eines Polymethylmethacrylat-Knochenzements, der Knochenzementapplikator aufweisend
A) eine Kartusche mit einem zylindrischen Innenraum,
B) einen Kartuschenkopf mit einer Austragsöffnung zum Austreiben des Knochenzements, wobei der Kartuschenkopf den zylindrischen Innenraum der Kartusche an einer Vorderseite der Kartusche bis auf die Austragsöffnung verschließt,
C) ein Mischorgan, das im zylindrischen Innenraum der Kartusche beweglich angeordnet ist und im Innenraum mit einem in den Innenraum geführten Mischstab bewegbar ist,
D) einen Austragskolben, der in der Kartusche angeordnet ist und der im zylindrischen Innenraum der Kartusche in Richtung der Austragsöffnung drückbar gelagert ist, wobei der Austragskolben eine äußere zylindrische Mantelfläche aufweist und wobei der Austragskolben mit seiner äußeren zylindrischen Mantelfläche zumindest bereichsweise an einer den zylindrischen Innenraum begrenzenden Innenwand der Kartusche anliegt, und
E) zumindest ein Klemmelement, das im Bereich einer der Vorderseite der Kartusche gegenüberliegenden Rückseite des zylindrischen Innenraums an der den zylindrischen Innenraum begrenzenden Innenwand der Kartusche angeordnet ist, wobei das zumindest eine Klemmelement aus der Innenwand der Kartusche in Richtung des zylindrischen Innenraums vorsteht, wobei die zylindrische Mantelfläche des Austragskolbens zumindest bereichsweise von dem zumindest einen Klemmelement in Richtung einer zentralen Zylinderachse des zylindrischen Innenraums elastisch deformierbar ist, so dass der Austragskolben mit dem zumindest einen Klemmelement an der Rückseite der Kartusche einklemmbar ist.

Bevorzugt ist der Austragskolben mit dem zumindest einen Klemmelement an der Rückseite der Kartusche lösbar einklemmbar. Lösbar bedeutet hierbei, dass der Austragskolben durch einen axialen Druck auf den Austragskolben aus der Klemmung durch das zumindest eine Klemmelement herausdrückbar ist. Axialer Druck bedeutet, dass der Kolben in Richtung der Vorderseite der Kartusche entlang oder parallel zur Zylinderachse des zylindrischen Innenraums gedrückt wird beziehungsweise drückbar ist.

Der zylindrische Innenraum der Kartusche hat bis auf die von dem zumindest einen Klemmkörper verursachten Asymmetrien eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Bevorzugt kann vorgesehen sein, dass der Mischstab durch den Austragskolben geführt ist und gegen den Austragskolben axial beweglich gelagert ist und bevorzugt auch drehbar beweglich gelagert ist.

Bevorzugt kann im zylindrischen Innenraum der Kartusche ein Zementpulver zur Herstellung des Knochenzements angeordnet sein.

Die zylindrische Mantelfläche des Austragskolbens ist zumindest bereichsweise von dem zumindest einen Klemmelement in Richtung einer Zylinderachse des zylindrischen Innenraums elastisch deformierbar, wenn der Austragskolben mit seiner zylindrischen Mantelfläche angrenzend zu dem zumindest einen Klemmelement im Innenraum der Kartusche angeordnet ist.

Gemäß einer bevorzugten Weiterbildung der vorliegenden Erfindung kann ein Zementpulver im zylindrischen Innenraum der Kartusche angeordnet sein. Der Knochenzementapplikator ist dann ein Prepacked-System, bei dem nur noch die Monomerflüssigkeit in den zylindrischen Innenraum der Kartusche zugeführt werden muss, um den Knochenzement im zylindrischen Innenraum mit Hilfe des Mischorgans zu mischen.

Es kann vorgesehen sein, dass das zumindest eine Klemmelement einteilig mit der Kartusche ausgebildet ist.

Hierdurch wird ein besonders einfacher und kostengünstig realisierbarer Aufbau erreicht. Zudem kann so verhindert werden, dass sich das zumindest eine Klemmelement gegen die Innenwand der Kartusche bewegen kann und so in seiner Funktion beeinträchtigt oder verändert wird.

Das zumindest eine Klemmelement ist bevorzugt einteilig beziehungsweise einstückig mit der Kartusche ausgebildet. Dadurch ist die Position des zumindest einen Klemmelements eindeutig definiert. Das zumindest eine Klemmelement kann dadurch in einfacher Weise zusammen mit der Kartusche in einem Kunststoffspritzgießprozess gefertigt werden.

Ferner kann vorgesehen sein, dass die Austragsöffnung in dem Kartuschenkopf mit einem lösbaren Verschluss verschlossen oder verschließbar ist, wobei bevorzugt ein Gewinde an dem Kartuschenkopf angeordnet ist, in das oder auf das der Verschluss mit einem zu dem Gewinde passenden Gegengewinde geschraubt ist oder schraubbar ist, um die Austragsöffnung zu verschließen.

Hierdurch kann der Knochenzement im zylindrischen Innenraum der Kartusche gemischt werden, ohne dass Teile der Ausgangskomponenten oder des Knochenzements austreten können.

Vorzugsweise ist der Verschluss für Gase durchlässig und für Flüssigkeiten und Pulver nicht durchlässig. Der Innenraum der Kartusche kann so durch den Verschluss hindurch mit Gasen sterilisiert werden.

Des Weiteren kann vorgesehen sein, dass der Außendurchmesser des Austragskolbens gleich oder kleiner ist als der Innendurchmesser des zylindrischen Innenraums der Kartusche, wobei bevorzugt am Außenumfang des Austragskolbens zumindest eine umlaufende Dichtung und/oder eine umlaufende Abstreiflippe angeordnet ist.

Hierdurch wird sichergestellt, dass der Austragskolben in dem Innenraum der Kartusche laufen kann. Mit der umlaufenden Dichtung und der Abstreiflippe kann sichergestellt werden, dass kein Knochenzement an dem Austragskolben vorbei an der Rückseite der Kartusche austritt und dass ein möglichst großer Anteil des Knochenzements aus der Kartusche ausgetrieben und genutzt werden kann.

Bevorzugt kann auch vorgesehen sein, dass der Innendurchmesser des zylindrischen Innenraums der Kartusche durch das zumindest eine Klemmelement im Bereich des zumindest einen Klemmelements reduziert ist, wobei vorzugsweise der Innendurchmesser so weit reduziert ist, dass der Austragskolben einen größeren Außendurchmesser aufweist als der durch das zumindest eine Klemmelement reduzierte Innendurchmesser.

Hierdurch wird erreicht, dass der Austragskolben im Innenraum der Kartusche einklemmbar ist, gleichzeitig aber noch im Innenraum der Kartusche unter Aufwendung entsprechender Kräfte gleiten kann.

Bei einer Weiterbildung der vorliegenden Erfindung kann vorgesehen sein, dass das zumindest eine Klemmelement ein umlaufender geschlossener Wulst oder Ausschnitte eines umlaufenden Wulsts oder zumindest zwei in axialer Richtung voneinander beabstandete umlaufende geschlossene Wulste oder zumindest zwei in axialer Richtung voneinander beabstandete Ausschnitte eines umlaufenden Wulsts sind.

Der Wulst oder der Ausschnitt kann dabei durchgehend oder auch mit Unterbrechungen geformt sein. Derartige Klemmelemente sind einfach zu fertigen und führen zu einer gleichmäßigen Klemmung des Austragskolbens. Zudem kann so eine aus jeder radialen Richtung wirkende Klemmung erfolgen, die einer möglichen Verkantung des Austragskolbens im Innenraum der Kartusche entgegenwirkt.

Ferner kann vorgesehen sein, dass das zumindest eine Klemmelement eine Fase an einer der Vorderseite der Kartusche zugewandten Vorderseite des zumindest einen Klemmelements und eine Fase an einer der Rückseite der Kartusche zugewandten Rückseite des zumindest einen Klemmelements hat, wobei die Fasen senkrecht zur Zylinderachse der Kartusche angeordnet sind.

Hiermit wird ein allzu plötzliches Lösen des Austragskolbens verhindert. Zudem kann der Austragskolben so leichter in die Klemmstellung gebracht werden und der Austragskolben zentriert sich selbstständig.

Es kann auch vorgesehen sein, dass zwei oder mehrere Klemmelemente axial bezüglich der Zylinderachse des Innenraums voneinander beabstandet an der Innenwand des zylindrischen Innenraums angeordnet sind.

Hiermit wird eine stabilere Klemmung des Austragskolbens erreicht und der Austragskolben löst sich nacheinander von den zumindest zwei axial beabstandeten Klemmelementen, so dass ein ruckartiges Lösen der Klemmung reduziert wird. Ferner wird dadurch die Klemmwirkung deutlich erhöht.

Gemäß einer bevorzugten Weiterbildung der vorliegenden Erfindung kann vorgesehen sein, dass eine der Innenwand des zylindrischen Innenraums zugewandte Außenwand des Austragskolbens eine axiale Erstreckung besitzt, die zumindest gleich groß der axialen Erstreckung des zumindest einen Klemmelements ist, wobei bevorzugt die axiale Erstreckung der Außenwand des Austragskolbens mindestens zweimal so groß ist, wie die axiale Erstreckung des zumindest einen Klemmelements.

Hierdurch wird sichergestellt, dass das zumindest eine Klemmelement die volle Klemmwirkung auf den Austragskolben entfalten kann.

Des Weiteren kann vorgesehen sein, dass eine der Innenwand des zylindrischen Innenraums zugewandte Außenwand des Austragskolbens als geschlossener Hohlzylinder ausgebildet ist.

Der Hohlzylinder kann als Ganzes radial elastisch komprimiert werden und so die Klemmwirkung verbessern.

Ferner kann vorgesehen sein, dass der von dem zumindest einen Klemmelement eingeklemmte Austragskolben durch Einwirkung einer Kraft, die längs der Zylinderachse der Kartusche gerichtet ist, lösbar ist und in Richtung der Vorderseite der Kartusche drückbar ist.

Hiermit wird sichergestellt, dass der Knochenzementapplikator mit einem unidirektionalen Antrieb verwendet werden kann, ohne dass weitere und andere Krafteinwirkungen notwendig sind.

Es kann vorgesehen sein, dass die äußere zylindrische Mantelfläche des Austragskolbens aus einem thermoplastischen Kunststoff besteht, wobei bevorzugt der thermoplastische Kunststoff ausgewählt ist aus zumindest einem der Kunststoffe Polyethylen, Polypropylen, Polyamid, Polyethylenterephthalat und Polybutylenterephthalat.

Thermoplastische Kunststoffe sind einfach zu fertigen und bieten die für die Klemmung notwendige Elastizität.

Auch die anderen Teile des Knochenzementapplikators können erfindungsgemäß aus einem der genannten Kunststoffe oder aus mehreren dieser Kunststoffe Polyethylen, Polypropylen, Polyamid, Polyethylenterephthalat und Polybutylenterephthalat bestehen.

Es kann auch vorgesehen sein, dass der Knochenzementapplikator einen Dichtungskolben aufweist, der mit dem Austragskolben verbindbar ist, wobei der Austragskolben die äußere zylindrische Mantelfläche und einen für Gase durchlässigen aber für das Zementpulver undurchlässigen Durchgang umfasst und wobei der Durchgang im Austragskolben mit dem Dichtungskolben dicht verschließbar ist, insbesondere durch Einschieben des Dichtungskolbens in eine in Richtung der Rückseite der Kartusche offene Öffnung des Austragskolbens verschließbar ist, wobei der Durchgang innerhalb der Öffnung des Austragskolbens angeordnet ist.

Hiermit kann der Innenraum der Kartusche, vorzugsweise mit dem Zementpulver darin, mit einem sterilisierenden Gas wie Ethylenoxid sterilisiert werden.

Die der vorliegenden Erfindung zugrundeliegenden Aufgaben werden auch gelöst durch ein Verfahren zur Herstellung eines Knochenzements, insbesondere eines pastenförmigen Polymethylmethacrylat-Knochenzements, wobei der Knochenzementteig aus einem Zementpulver und einer Monomerflüssigkeit mit einem erfindungsgemäßen Knochenzementapplikator hergestellt wird, gekennzeichnet durch die folgenden nacheinander ablaufenden Schritte:
A) Einfüllen des Zementpulvers und der Monomerflüssigkeit in den zylindrischen Innenraum der Kartusche oder der Monomerflüssigkeit zu dem Zementpulver im zylindrischen Innenraum der Kartusche,
B) Verschließen der Kartusche durch Eindrücken des Austragskolbens in den zylindrischen Innenraum der Kartusche,
C) Einklemmen des Austragskolbens an der Rückseite der Kartusche mit dem zumindest einen Klemmelement,
D) Mischen des Knochenzements im zylindrischen Innenraum der Kartusche durch Bewegen des Mischorgans im zylindrischen Innenraum der Kartusche,
E) Eindrücken der Austragskolbens in Richtung der Vorderseite der Kartusche, wobei sich dabei die Klemmung des zumindest einen Klemmelements löst, und
F) Auspressen des Knochenzements durch die Austragsöffnung aus der Kartusche durch Vortreiben des Austragskolbens im Innenraum der Kartusche in Richtung des Kartuschenkopfs.

Das Verfahren ist bevorzugt kein medizinisches Verfahren. Das Verfahren hat besonders bevorzugt keinerlei Wechselwirkung mit einem menschlichen oder tierischen Körper. Der Knochenzement kann nach dem Ausfließen aus der Austragsöffnung durch ein an der Austragsöffnung befestigtes Austragsrohr gepresst werden oder in einen Behälter gefüllt werden, bevor er zum Zementieren verwendet wird.

Bei dem erfindungsgemäßen Verfahren kann vorgesehen sein, dass das Zementpulver vor der Monomerflüssigkeit in den Innenraum der Kartusche gefüllt wird oder bereits in dem zylindrischen Innenraum der Kartusche enthalten ist.

Hierdurch kann der Innenraum der Kartusche besser genutzt und vom Volumen kleiner gestaltet werden und es wird eine effizientere Mischung des Knochenzements gewährleistet.

Ferner kann vorgesehen sein, dass beim Einklemmen des Austragskolbens in Schritt C) der Austragskolben in Richtung der Zylinderachse des zylindrischen Innenraums der Kartusche elastisch deformiert wird, wobei vorzugsweise ein rohrförmiger Fortsatz an der Rückseite des Austragskolbens, dessen Außenfläche zumindest bereichsweise durch die äußere zylindrische Mantelfläche des Austragskolbens begrenzt ist, von dem zumindest einen Klemmelement in Richtung der Zylinderachse des zylindrischen Innenraums der Kartusche gedrückt wird.

Hiermit wird eine besonders effektive und gleichzeitig gut zu lösende Klemmung erreicht.

Des Weiteren kann vorgesehen sein, dass in Schritt D) das Mischorgan mit dem Mischstab bewegt wird und anschließend mit dem Mischstab gegen eine der Vorderseite der Kartusche zugewandte Vorderseite des Austragskolbens gezogen wird und anschließend der Mischstab abgebrochen wird.

Hierdurch stört das Mischorgan und der Mischstab nicht beim Auspressen des Knochenzements.

Es kann auch vorgesehen sein, dass vor Schritt E) der Knochenzementapplikator in eine Auspressvorrichtung mit einem axial beweglichen und antreibbaren Stößel eingesetzt wird, wobei in Schritt E) und in Schritt F) der Austragskolben mit dem Stößel angetrieben wird.

Hierdurch kann eine kraftvolle Bewegung des Austragskolbens zum Lösen der Klemmung und zum Auspressen des Knochenzements verwendet werden.

Des Weiteren kann vorgesehen sein, dass die Austragsöffnung verschlossen ist und vor Schritt E) oder F) geöffnet wird.

Hiermit kann verhindert werden, dass beim Mischen des Knochenzements Teile davon nach außen dringen.

Der Erfindung liegt die überraschende Erkenntnis zugrunde, dass es durch die Verwendung von einfachen Klemmelementen gelingt, den Austragskolben zuverlässig aber lösbar gegen die Kartusche zu fixieren und dabei die Fixierungsposition einen gewissen Spielraum hat, der Austragskolben also über eine gewisse Wegstrecke in dem Innenraum der Kartusche fixiert ist. Dadurch kann der Knochenzement im Innenraum der Kartusche gemischt werden, ohne dass sich dabei der Austragskolben löst. Zum Lösen des Austragskolbens kann dieser einfach über die Klemmposition hinaus in den Innenraum der Kartusche eingedrückt werden. Aufgrund der elastischen Deformation, die das zumindest eine Klemmelement auf den Austragskolben bewirkt, kann nach dem Lösen der Klemmung sich der Austragskolben wieder in seinen entspannten Ursprungszustand umformen und dadurch dicht und bündig im Innenraum der Kartusche bewegt werden, um den Knochenzement auszupressen. Der Knochenzementapplikator kann so kostengünstig aufgebaut werden.

Bei axialer Krafteinwirkung einer mechanischen Auspressvorrichtung auf den Austragskolben in Richtung des Kartuschenkopfs wird der Austragskolben aus seinem geklemmten Sitz herausgedrückt und in Richtung des Kartuschenkopfs verschoben. Dadurch, dass das zumindest eine Klemmelement nur an der Innenseite der Kartusche angebracht ist und dass der Austragskolben keine Vorsprünge besitzt, die eine größeren Durchmesser als der Innendurchmesser der Kartusche haben, kann der Austragskolben nach erfolgter Lösung vom zumindest einen Klemmelement der Kartusche in Richtung des Kartuschenkopfs verschoben werden, ohne dass Bremseffekte durch die Bestandteile der Klemmung erfolgen können.

Die Vorteile erfindungsgemäßer Knochenzementapplikatoren und Verfahren beruhen grundsätzlich auch darauf, dass die an sich bekannte lineare Bewegungen so genutzt werden, um den Austragskolben zu lösen und um den Inhalt auszutreiben. Der Knochenzementapplikator kann als hygienisches Wegwerfprodukt verwendet werden, da er sehr weitgehend aus Kunststoff gefertigt werden kann und weil alle Teile einschließlich der Innenräume und des Zementpulvers mit Hilfe von Ethylenoxid sterilisierbar sind.

Ein beispielhafter und bevorzugter erfindungsgemäßer Knochenzementapplikator für die Vermischung und zum Austragen von Polymethylmethacrylat-Knochenzement kann zusammengesetzt sein aus einer hohlzylinderförmigen Kartusche, einem lösbaren Verschluss in einem Kartuschenkopf, einem Mischstabs mit einem daran befestigten Mischorgan und einem Austragskolben. Dabei kann vorgesehen sein, dass an der Innenseite der Kartusche zumindest ein Klemmelement angebracht ist, wobei der Innendurchmesser des Klemmelements kleiner ist als der Innendurchmesser der Kartusche, der Austragskolben durch eine zylinderförmige Außenwand begrenzt ist, wobei der Außendurchmesser des Austragskolbens gleich oder kleiner ist als der Innendurchmesser der Kartusche, die Außenwand des Austragskolbens elastisch in Richtung der Längsachse des Austragskolbens deformierbar ist, und der Austragskolben so in der Kartusche angeordnet ist, dass das mindestens eine Klemmelement die Außenwand des Austragskolbens in Richtung der Längsachse des Austragskolbens elastisch verformt, wobei der Austragskolben mit der Kartusche verklemmt.

Der Austragskolben kann einteilig oder auch zweiteilig gemäß der Lehre des Patents DE 10 2009 031 178 B3 gestaltet sein, auf dessen Lehre und Inhalt hiermit vollinhaltlich Bezug genommen wird. Weiterhin beziehungsweise Alternativ ist es auch möglich, den Mischstab durch den Kartuschenkopf zu führen und als Austragskolben einen einfachen Kolben mit elastisch deformierbarer Außenwand zu verwenden, der mit dem zumindest einen Klemmelement der Kartusche verklemmt werden kann.

Im Folgenden werden weitere Ausführungsbeispiele der Erfindung anhand von dreizehn schematisch dargestellten Figuren erläutert, ohne jedoch dabei die Erfindung zu beschränken. Dabei zeigt:
Figur 1: eine schematische Querschnittansicht eines ersten beispielhaften erfindungsgemäßen Knochenzementapplikators zum Mischen und Austragen eines Knochenzements mit nicht arretiertem Austragskolben;
Figur 2: eine schematische perspektivische Querschnittansicht des Knochenzementapplikators nach Figur 1 mit arretiertem Austragskolben;
Figur 3: eine schematische perspektivische Querschnittansicht des Knochenzementapplikators nach den Figuren 1 und 2 bei Auspressen mit einer Auspressvorrichtung;
Figur 4: eine schematische perspektivische Außenansicht des Knochenzementapplikators nach den Figuren 1 bis 3;
Figur 5: eine schematische perspektivische Querschnittansicht des Knochenzementapplikators nach Figur 4;
Figur 6: die Kartusche und den Austragskolben des Knochenzementapplikators nach Figur 4 als einzelne schematische perspektivische Querschnittansichten;
Figur 7: eine Ausschnittvergrößerung der Figur 1 im Bereich des nicht arretierten Austragskolbens;
Figur 8: eine Ausschnittvergrößerung der Figur 2 im Bereich des arretierten Austragskolbens;
Figur 9: eine Ausschnittvergrößerung der Figur 3 im Bereich des mit der Auspressvorrichtung angetriebenen Austragskolbens;
Figur 10: einen Ausschnitt einer schematischen Querschnittansicht eines zweiten beispielhaften erfindungsgemäßen Knochenzementapplikators zum Mischen und Austragen eines Knochenzements mit arretiertem Austragskolben analog Figur 8;
Figur 11: einen Ausschnitt einer schematischen Querschnittansicht eines dritten beispielhaften erfindungsgemäßen Knochenzementapplikators zum Mischen und Austragen eines Knochenzements mit arretiertem Austragskolben analog Figur 8;
Figur 12: die Kartusche des dritten bespielhaften Knochenzementapplikators nach Figur 11 als einzelne schematische perspektivische Detail- und Querschnittansicht; und
Figur 13: einen Ausschnitt einer schematischen Querschnittansicht eines vierten beispielhaften erfindungsgemäßen Knochenzementapplikators zum Mischen und Austragen eines Knochenzements mit nicht arretiertem Austragskolben analog Figur 7.

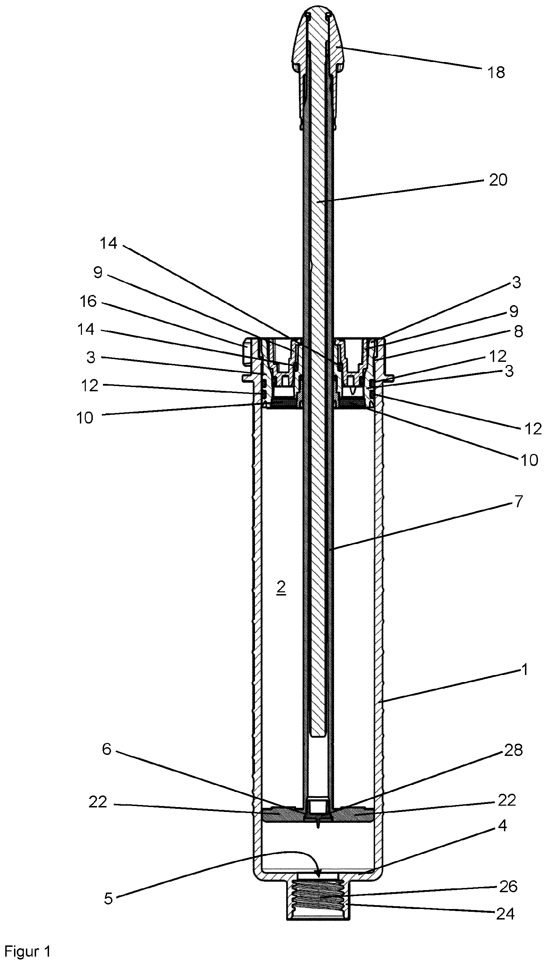

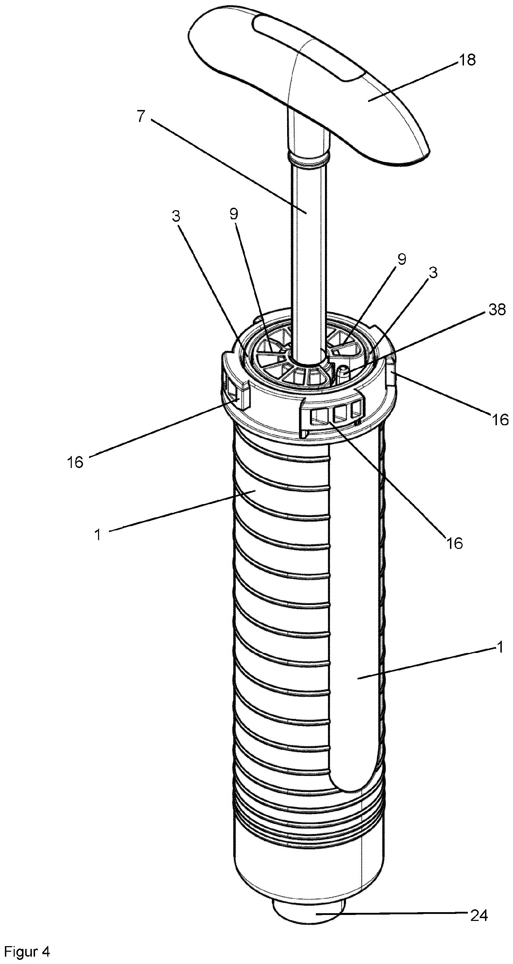

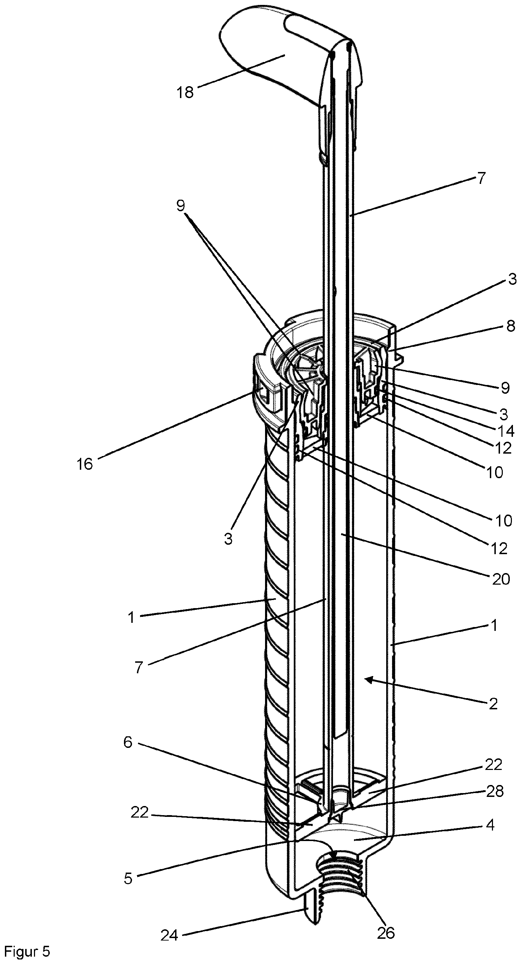

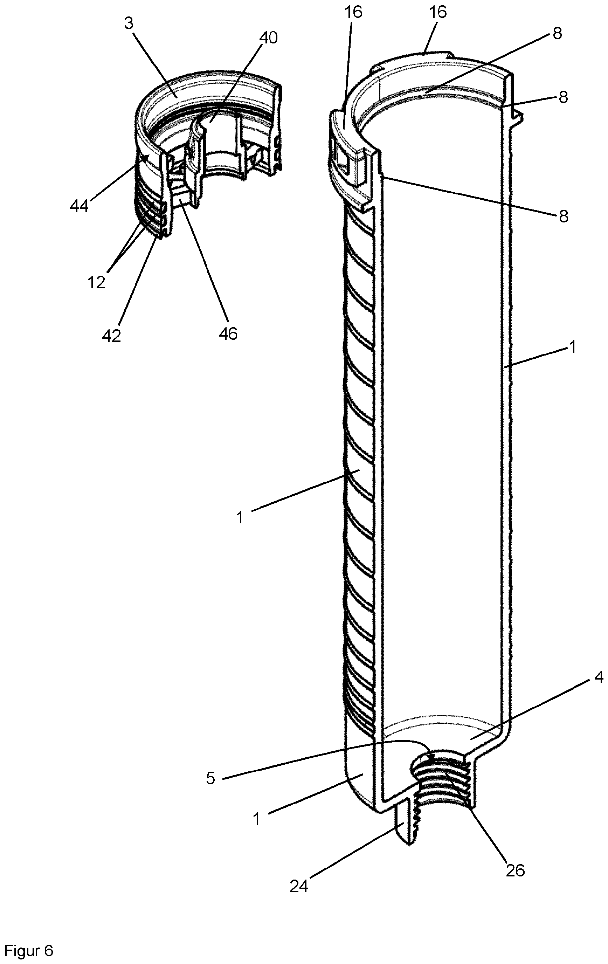

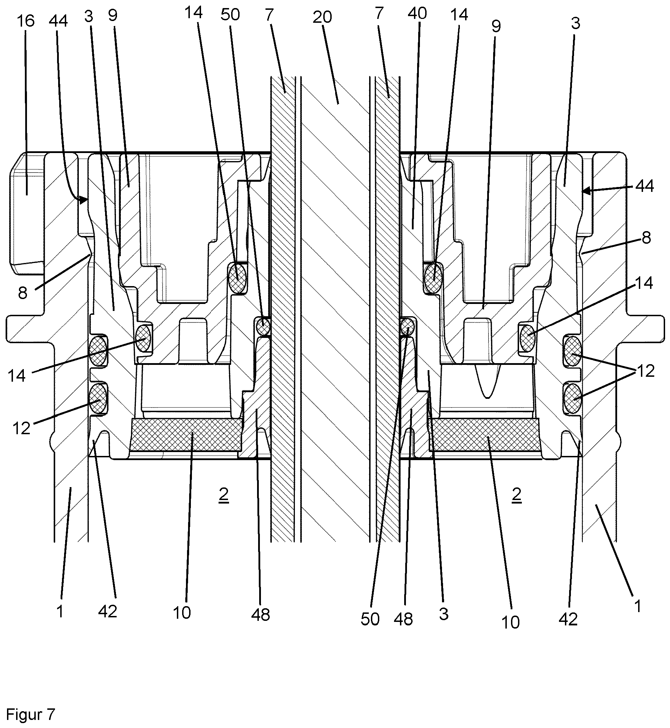

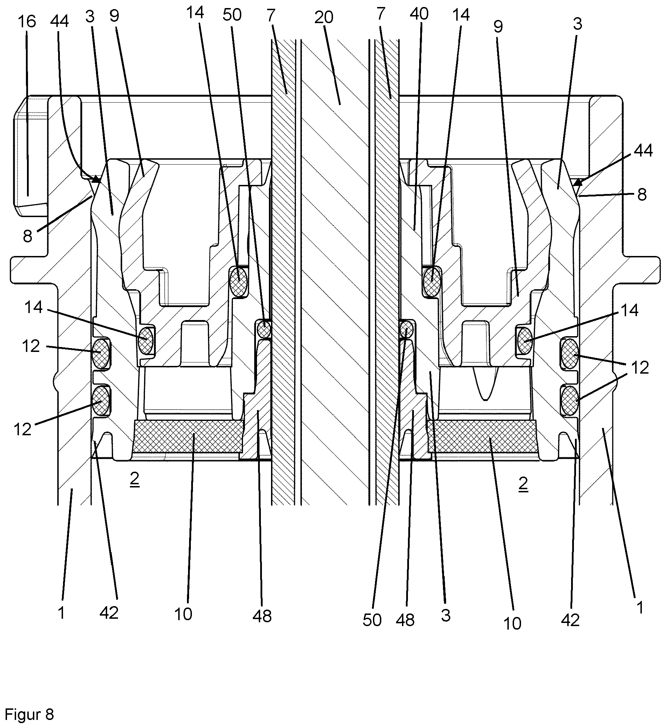

In den Figuren 1 bis 9 sind Abbildungen eines ersten erfindungsgemäßen Knochenzementapplikators gezeigt. Die Figuren 1 bis 5 zeigen verschiedene schematische Gesamtansichten des ersten beispielhaften erfindungsgemäßen Knochenzementapplikators. Figur 6 zeigt Querschnittansichten zweier Teile des Knochenzementapplikators und die Figuren 7 bis 9 zeigen Ausschnittvergrößerungen der Figuren 1 bis 3 in Form von schematischen Querschnittansichten als Detailansichten durch einen Bereich des erfindungsgemäßen Knochenzementapplikators. In allen Figuren ist die Vorderseite des Knochenzementapplikators unten und die Rückseite des Knochenzementapplikators oben angeordnet.

Gemäß dem ersten Ausführungsbeispiel der vorliegenden Erfindung kann der Knochenzementapplikator eine röhrenförmigen Kartusche 1 aus einem Kunststoff mit einem zylindrischen Innenraum 2 aufweisen. Im zylindrischen Innenraum 2 der Kartusche 1 kann ein in axialer Richtung beweglich gelagerter Austragskolben 3 angeordnet sein. An einer Vorderseite der Kartusche (in den Figuren unten) kann der zylindrische Innenraum 2 durch einen Kartuschenkopf 4 verschlossen sein, der einteilig mit der Kartusche ausgeführt sein kann. Alternativ kann der Kartuschenkopf 4 auch als separates Teil auf die Kartusche 1 aufgeschraubt sein. In dem Kartuschenkopf 4 kann eine Austragsöffnung 5 angeordnet sein, durch die ein Knochenzement (in den Figuren nicht gezeigt), der im zylindrischen Innenraum 2 der Kartusche 1 enthalten sein kann beziehungsweise gemischt werden kann, mit dem Austragskolben 3 aus dem Innenraum 2 ausgetrieben werden kann. Die Austragsöffnung 5 kann verschließbar oder verschlossen sein. Im zylindrischen Innenraum 2 der Kartusche 1 kann ein Zementpulver (nicht gezeigt) als Ausgangskomponente des herzustellenden Knochenzements angeordnet sein. Eine Monomerflüssigkeit (nicht gezeigt) als zweite Ausgangskomponente kann zu dem Zementpulver in den zylindrischen Innenraum 2 gefüllt werden, um den Knochenzement in dem Innenraum zu mischen.

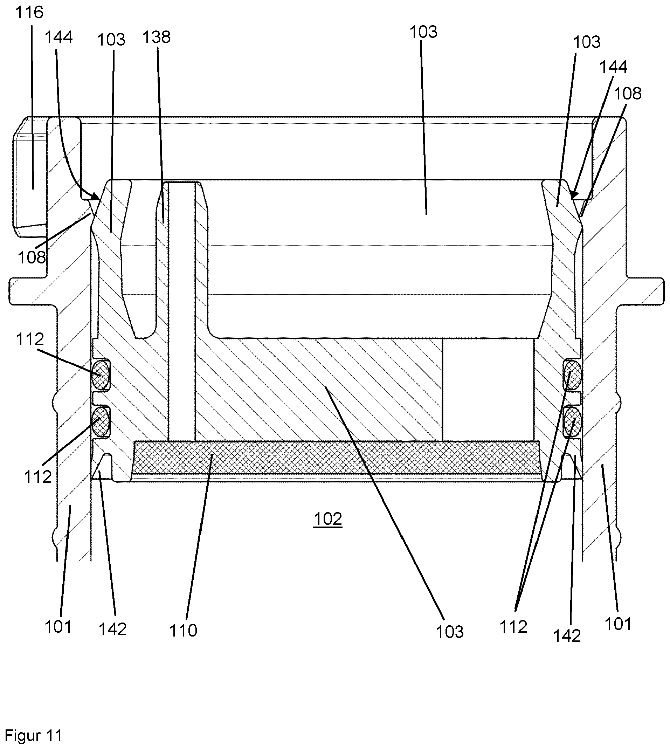

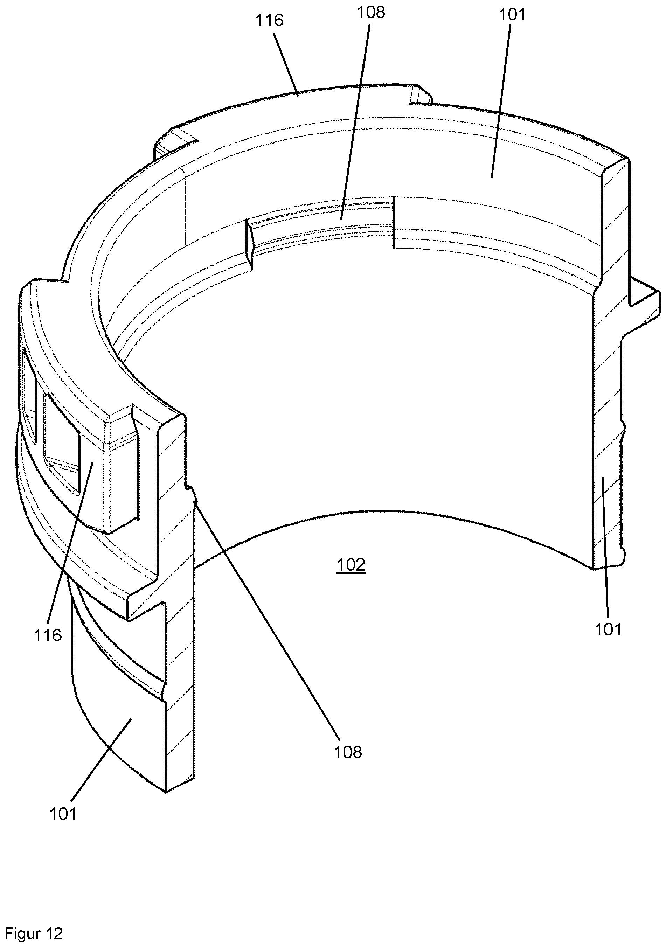

Zum Durchmischen des Inhalts des Innenraums 2 kann ein Mischorgan 6 in dem zylindrischen Innenraum 2 beweglich angeordnet sein. Das Mischorgan 6 kann mit einem Mischstab 7 in dem zylindrischen Innenraum 2 axial bewegt und um die Achse des Mischstabs 7 gedreht werden. Der Mischstab 7 kann durch eine Durchführung im Austragskolben 3 in den Innenraum 2 der Kartusche 1 geführt sein. Alternativ könnte der Mischstab 7 auch durch den Kartuschenkopf geführt werden (siehe das dritte Ausführungsbeispiel nach den Figuren 11 und 12).

An der Innenwand der Kartusche 1 kann im Bereich des rückseitigen Endes der Kartusche 1 (in den Figuren oben) eine vorspringende umlaufende Leiste als Klemmelement 8 zum Festklemmen des Austragskolbens 3 angeordnet sein. Wenn der Austragskolben 3 in das rückseitige Ende der Kartusche 1 eingedrückt wird, kann das Klemmelement 8 einen radialen Druck auf den Austragskolben 3 ausüben. Durch den radialen Druck kann der Austragskolben 3 elastisch deformiert werden. Die elastische Deformation des Austragskolbens 3 kann eine Klemmung des Austragskolbens 3 in der Höhe des Klemmelements 8 bewirken.

In der Rückseite des Austragskolbens 3 kann ein Dichtungskolben 9 angeordnet sein, mit dem ein Durchgang durch den Austragskolben 3 abgedichtet werden kann. In dem Durchgang durch den Austragskolben 3 kann ein für Gase durchlässiger aber für das Zementpulver undurchlässiger Porenfilter 10 angeordnet sein. Durch den Durchgang im Austragskolben 3 und gegebenenfalls durch den Porenfilter 10 kann der Innenraum 2 der Kartusche 1 und dessen Inhalt mit Hilfe eines sterilisierenden Gases wie Ethylenoxid sterilisiert werden. Der Dichtungskolben 9 kann anschließend eingeschoben werden, um den Durchgang abzudichten. Bei abgedichtetem Durchgang kann der Innenraum 2 der Kartusche 1 evakuiert werden, um den Knochenzement unter Vakuum mischen zu können.

Um eine Evakuierung des Innenraums 2 zu ermöglichen, können an der Außenwandung des Austragskolbens 3 zwei umlaufende Dichtungen 12 aus Gummi angeordnet sein, mit dem der Austragskolben 3 gegen die Innenwand der Kartusche 1 und damit den zylindrischen Innenraum 2 abgedichtet ist. Ebenso kann der Dichtungskolben 9 gegen den Austragskolben 3 mit einer umlaufenden Dichtung 14 abgedichtet sein. Ferner kann der Austragskolben 3 gegen den Mischstab 7 mit einer umlaufenden Dichtung 50 abgedichtet sein (siehe die Detailansichten nach den Figuren 7, 8 und 9).

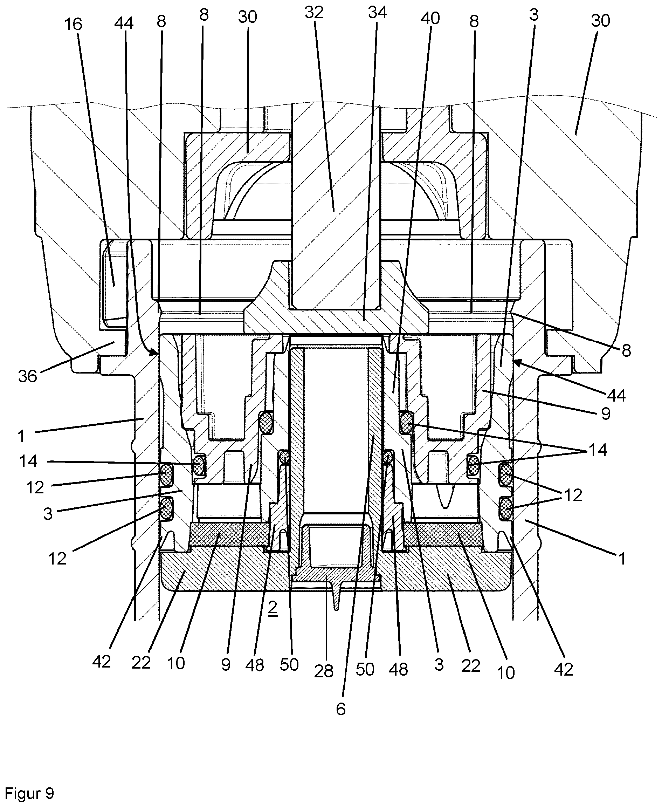

An der Rückseite der Kartusche 1 können außen mehrere Befestigungsmittel 16 angeordnet sein, an denen einen Auspressvorrichtung 30 befestigt werden kann (siehe Figur 9).

Der Mischstab 7 kann in einem Griff 18 enden, mit dem der Mischstab 7 und damit das Mischorgan 6 im Innenraum 2 der Kartusche 1 bewegt werden kann. Ein Kern 20 kann im Inneren des Mischstabs 7 angeordnet sein, der den Mischstab 7 mechanisch stabilisiert. Der Mischstab 7 kann in einer Durchführung durch den Austragskolben 3 beweglich gelagert sein. Der Mischstab 7 kann durch die Durchführung durch den Austragskolben 3 axial bewegt werden und vorzugsweise auch in der Durchführung gedreht werden. Wenn das Mischorgan 6 starr mit dem Mischstab 7 verbunden ist, kann dementsprechend das Mischorgan 6 im Innenraum 2 der Kartusche 1 axial bewegt werden und vorzugsweise auch gedreht werden. Hierdurch ist der Inhalt der Kartusche 1 mit dem Mischorgan 6 manuell mischbar. Das Mischorgan 6 kann mehrere Mischflügel 22 aufweisen, die der Durchmischung des Innenraums 2 dienen. Das Mischorgan 6 kann einen umlaufenden Ring aufweisen, der an der Wandung des zylindrischen Innenraums 2 anliegt. Dadurch können alle Bereiche des Innenraums 2 mit dem Mischorgan 6 erreicht werden und so eine vollständige Durchmischung des Knochenzements im Innenraum 2 sichergestellt werden.

Am Kartuschenkopf 4 kann ein Stutzen 24 angeordnet sein. Die Austragsöffnung 5 kann im Inneren des Stutzens 24 angeordnet sein. In dem Stutzen 24 kann ein Innengewinde 26 zur Befestigung eines Austragsrohrs (nicht gezeigt) angeordnet sein. Über das Austragsrohr kann der Knochenzement appliziert werden oder in eine Schale zur späteren Anwendung geleitet werden. Die Austragsöffnung 5 kann mit einem Verschluss (nicht gezeigt) verschlossen sein. Beispielsweise kann ein schraubbarer Verschluss mit einem Außengewinde in das Innengewinde 26 des Stutzens 24 eingeschraubt sein. Der Verschluss kann entfernt werden, wenn der Knochenzement fertig gemischt wurde.

Der Mischstab 7 kann an seiner Vorderseite mit einem Stopfen 28 verschlossen sein, damit der Knochenzement nicht in den Mischstab 7 eindringt.

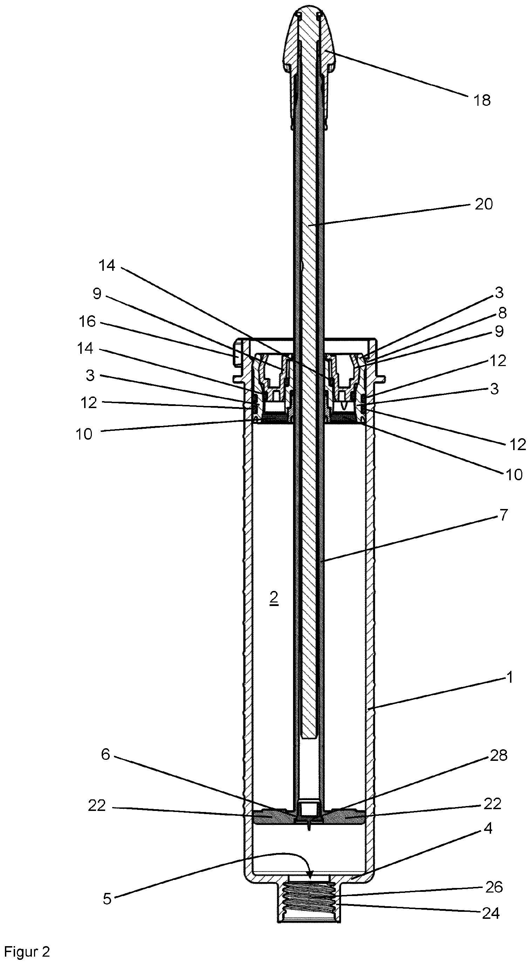

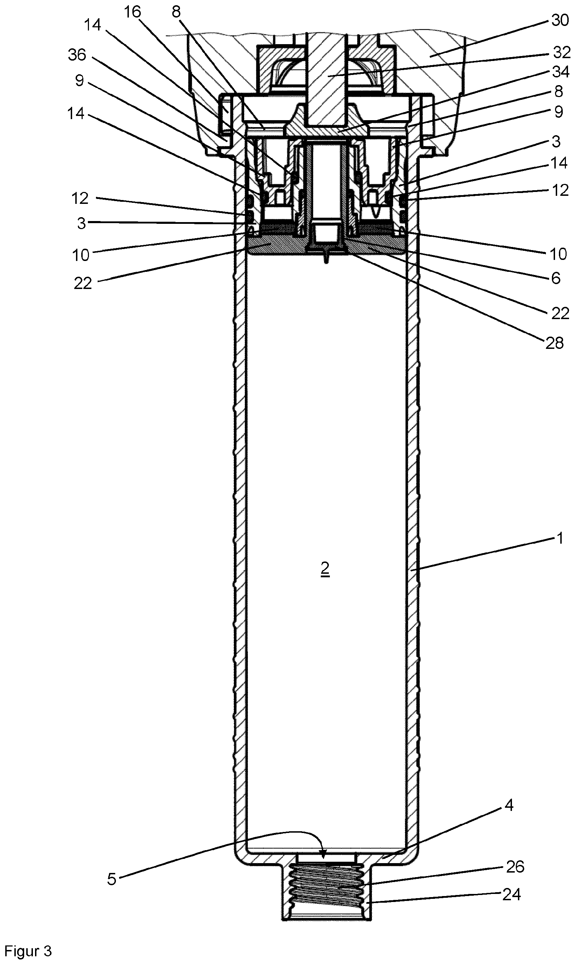

Zum Lösen der Klemmung des Austragskolbens 3 mit dem Klemmelement 8 (siehe Figuren 2, 5 und 8) kann eine Auspressvorrichtung 30 verwendet werden, deren vorderer Teil in Figur 3 dargestellt ist. Solche Auspressvorrichtungen 30 sind aus dem Stand der Technik gut bekannt und werden daher vorliegend nicht im Detail beschrieben. Die Auspressvorrichtung 30 kann eine axial vortreibbare Schubstange 32 aufweisen. An der Spitze der Schubstange 32 kann ein Teller 34 angeordnet sein, mit dem der Austragskolben 3 in der Kartusche 1 axial in Richtung des Kartuschenkopfs 4 vortreibbar ist. Die Auspressvorrichtung 30 kann über Gegenbefestigungsmittel 36 an den Befestigungsmitteln 16 der Kartusche 1 befestigt werden, beispielsweise nach Art eines Bajonett-Verschlusses. Mit der Auspressvorrichtung 30 kann nicht nur die Klemmung (siehe Figuren 2, 5 und 8) gelöst werden, sondern auch der Austragskolben 3 weiter in Richtung des Kartuschenkopfs 4 getrieben werden und dabei der Inhalte der Kartusche 1 (also der Knochenzement) durch die Austragsöffnung 5 aus dem Innenraum der Kartusche 1 ausgetrieben werden. Die Auspressvorrichtung 30 kann nach Art einer Kartuschenpistole manuell angetrieben werden oder auch mit einem Gasdruck oder elektrisch angetrieben werden.

Zur Evakuierung des Innenraums 2 der Kartusche 1 kann an dem Austragskolben 3 beziehungsweise an dem Dichtungskolben 9 ein Vakuumanschluss 38 angeordnet sein. Durch den Vakuumanschluss 38 kann Gas aus dem Innenraum 2 der Kartusche 1 evakuiert werden, wenn der Dichtungskolben 9 den Durchgang durch den Austragskolben 3 abdichtet.

Zur Führung des Mischstabs 7 kann der Austragskolben 3 in einem zentralen Bereich als eine Hülse 40 ausgeformt sein. An seiner Verbindung zur Innenwand der Kartusche 1 und/oder zum Mischstab 7 kann der Austragskolben 3 Abstreiflippen 42 aufweisen, mit denen verhindert werden kann, dass der Knochenzement zwischen dem Austragskolben 3 und der Innenwand der Kartusche 1 und zwischen dem Austragskolben 3 und dem Mischstab 7 vordringen und austreten kann.

Bei einer Klemmung des Austragskolbens 3 mit dem Klemmelement 8 kann das Klemmelement 8 auf eine zylindrische äußere Mantelfläche 44 des Austragskolbens 3 drücken. Das umlaufende Klemmelement 8 kann dabei den Austragskolben 3 radial komprimieren. Der Austragskolben 3 kann dabei durch das Klemmelement 8 elastisch verformt werden, was die Klemmung und damit ein Arretieren des Austragskolbens bewirkt. Beim Lösen der Klemmung mit der Auspressvorrichtung 30 kann die Klemmung gelöst werden und die elastische Deformation des Austragskolbens 3 kann wieder rückgängig gemacht werden, indem sich der Austragskolben 3 beziehungsweise die elastisch deformierten Bereiche des Austragskolbens 3, nämlich die Mantelfläche 44, wieder entspannen (siehe Figuren 3und 9).

Die Hülse 40 kann über Streben 46 mit den äußeren Bereichen des Austragskolbens 3, umfassend die Mantelfläche 44 des Austragskolbens 3, verbunden sein. Zwischen den Streben 46 kann der Durchgang zum Evakuieren durch den Austragskolben 3 geformt sein. Der Porenfilter 10 kann als Ringscheibe ausgeformt sein und auf den Streben 46 aufliegen. Um ein Herausfallen des Porenfilters 10 zu verhindern, kann der Austragskolben 3 eine Einsteckhülse 48 aufweisen, mit der der Porenfilter 10 gesichert ist und durch den der Mischstab 7 geführt ist.

Der erste beispielhafte Knochenzementapplikator kann erfindungsgemäß wie folgt angewendet werden. Ein Zementpulver kann in dem Innenraum 2 der Kartusche 1 enthalten sein oder es wird in den Innenraum 2 eingefüllt. Die Austragsöffnung 5 ist vorzugsweise mit einem Verschluss verschlossen. Die Monomerflüssigkeit kann anschließend zugeführt werden. Der Dichtungskolben 9 kann zur Abdichtung in den Austragskolben 3 eingeschoben werden. Der Austragskolben 3 kann soweit in die Kartusche 1 eingeschoben werden, dass das Klemmelement 8 den Austragskolben 3 an der Mantelfläche 44 elastisch deformiert und so den Austragskolben 3 einklemmt. Über den Vakuumanschluss 38 kann im Innenraum 2 der Kartusche 1 ein Vakuum erzeugt werden. Das Zementpulver kann mit der Monomerflüssigkeit durch Bewegen des Mischorgans 6 im Innenraum 2 gemischt werden. Dabei bewegt sich der Austragskolben 3 nicht mit dem Mischstab 7, da er durch die Klemmung mit dem Klemmelement 8 arretiert ist. Das Mischorgan 6 kann mit dem Mischstab 7 an den arretierten Austragskolben 3 gezogen werden und der Mischstab 7 kann abgebrochen werden.

Anschließend kann der Knochenzementapplikator in die Auspressvorrichtung 30 eingesetzt werden. Durch Vortreiben der Schubstange 32 kann der Austragskolben 3 angetrieben werden. Dabei kann zunächst die Klemmung gelöst werden und so der Austragskolben 3 von dem Klemmelement 8 gelöst werden. Der Verschluss kann aus der Austragsöffnung 5 entfernt werden und es kann ein Austragsrohr an dem Innengewinde 26 befestigt werden. Der Knochenzement kann durch weiteres Vortreiben des Austragskolbens 3 im Innenraum 2 der Kartusche 1 in Richtung des Kartuschenkopfs 4 aus der Austragsöffnung 5 ausgedrückt werden. Dort kann der Knochenzementteig durch das Austragsrohr ausgepresst werden.

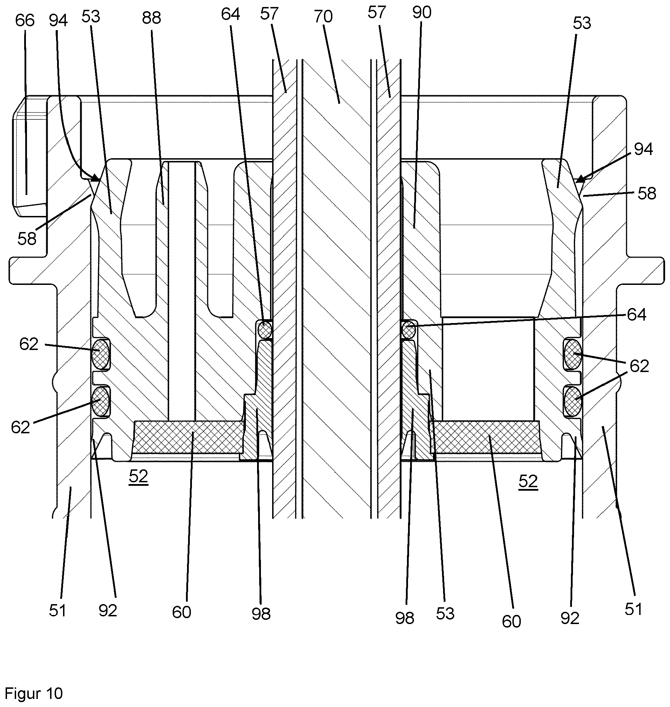

In Figur 10 ist ein Ausschnitt eines zweiten erfindungsgemäßen Knochenzementapplikators als Querschnittansicht gezeigt. In der Figur ist die Vorderseite des Knochenzementapplikators unten und die Rückseite des Knochenzementapplikators oben angeordnet. Der zweite erfindungsgemäße Knochenzementapplikator gleicht dem ersten nach den Figuren 1 bis 9, bis auf den einteiligen Aufbau des Austragskolbens 53.

Gemäß dem zweiten Ausführungsbeispiel der vorliegenden Erfindung kann der Knochenzementapplikator eine röhrenförmigen Kartusche 51 aus einem Kunststoff mit einem zylindrischen Innenraum 52 aufweisen. Im zylindrischen Innenraum 52 der Kartusche 51 kann der in axialer Richtung beweglich gelagerter Austragskolben 53 angeordnet sein. An einer Vorderseite der Kartusche (in Figur 10 nicht zu sehen) kann der zylindrische Innenraum 52 durch einen Kartuschenkopf verschlossen sein. In dem Kartuschenkopf kann eine Austragsöffnung angeordnet sein, durch die ein Knochenzement (in Figur 10 nicht gezeigt), der im zylindrischen Innenraum 52 der Kartusche 51 enthalten sein kann beziehungsweise gemischt werden kann, mit dem Austragskolben 53 aus dem Innenraum 52 ausgetrieben werden kann. Die Austragsöffnung kann verschließbar oder verschlossen sein. Im zylindrischen Innenraum 52 der Kartusche 51 kann ein Zementpulver (nicht gezeigt) als Ausgangskomponente des herzustellenden Knochenzements angeordnet sein. Eine Monomerflüssigkeit (nicht gezeigt) als zweite Ausgangskomponente kann zu dem Zementpulver in den zylindrischen Innenraum 52 gefüllt werden, um den Knochenzement in dem Innenraum zu mischen.

Zum Durchmischen des Inhalts des Innenraums 52 kann ein Mischorgan (in Figur 10 nicht zu sehen, aber analog dem ersten Ausführungsbeispiel) in dem zylindrischen Innenraum 52 beweglich angeordnet sein. Das Mischorgan kann mit einem Mischstab 57 in dem zylindrischen Innenraum 52 axial bewegt und um die Achse des Mischstabs 57 gedreht werden. Der Mischstab 57 kann durch eine Durchführung im Austragskolben 53 in den Innenraum 52 der Kartusche 51 geführt sein.

An der Innenwand der Kartusche 51 kann im Bereich des rückseitigen Endes der Kartusche 51 (in Figur 10 oben) eine vorspringende Leiste als Klemmelement 58 zum Festklemmen des Austragskolbens 53 angeordnet sein. Wenn der Austragskolben 53 in das rückseitige Ende der Kartusche 51 eingedrückt wird, kann das Klemmelement 58 einen radialen Druck auf den Austragskolben 53 ausüben. Durch den radialen Druck kann der Austragskolben 53 elastisch deformiert werden. Die elastische Deformation des Austragskolbens 53 kann eine Klemmung des Austragskolbens 53 in der Höhe des Klemmelements 58 bewirken.

In einem Durchgang durch den Austragskolben 53 kann ein für Gase durchlässiger aber für das Zementpulver undurchlässiger Porenfilter 60 angeordnet sein. Durch den Durchgang im Austragskolben 53 und gegebenenfalls durch den Porenfilter 60 kann der Innenraum 52 der Kartusche 51 und dessen Inhalt mit Hilfe eines sterilisierenden Gases wie Ethylenoxid sterilisiert werden und der Innenraum 52 der Kartusche 51 evakuiert werden, um den Knochenzement unter Vakuum mischen zu können.

Um eine Evakuierung des Innenraums 52 zu ermöglichen, können an der Außenwandung des Austragskolbens 53 zwei umlaufende Dichtungen 62 aus Gummi angeordnet sein, mit dem der Austragskolben 53 gegen die Innenwand der Kartusche 51 und damit den zylindrischen Innenraum 52 abgedichtet ist. Ebenso kann der Mischstab 57 gegen den Austragskolben 53 mit einer umlaufenden Dichtung 64 abgedichtet sein.

An der Rückseite der Kartusche 51 können außen mehrere Befestigungsmittel 66 angeordnet sein, an denen einen Auspressvorrichtung (nicht gezeigt aber analog Figur 3) befestigt werden kann.

Mit dem Mischstab 57 kann das Mischorgan im Innenraum 52 der Kartusche 51 bewegt werden kann. Ein Kern 70 kann im Inneren des Mischstabs 57 angeordnet sein, der den Mischstab 57 mechanisch stabilisiert. Der Mischstab 57 kann in einer Durchführung durch den Austragskolben 53 beweglich gelagert sein. Der Mischstab 57 kann durch die Durchführung durch den Austragskolben 53 axial bewegt werden und vorzugsweise auch in der Durchführung gedreht werden. Wenn das Mischorgan starr mit dem Mischstab 57 verbunden ist, kann dementsprechend das Mischorgan im Innenraum 52 der Kartusche 51 axial bewegt werden und vorzugsweise auch gedreht werden. Hierdurch ist der Inhalt der Kartusche 51 mit dem Mischorgan manuell mischbar.

Zum Lösen der Klemmung des Austragskolbens 53 mit dem Klemmelement 58 (siehe Figur 10) kann eine Auspressvorrichtung analog Figur 3 verwendet werden. Mit der Auspressvorrichtung 30 kann nicht nur die Klemmung (siehe Figur 10) gelöst werden, sondern auch der Austragskolben 53 weiter in Richtung des Kartuschenkopfs getrieben werden und dabei der Inhalte der Kartusche 51 (also der Knochenzement) durch die Austragsöffnung aus dem Innenraum der Kartusche 51 ausgetrieben werden.

Zur Evakuierung des Innenraums 52 der Kartusche 51 kann an dem Austragskolben 53 ein Vakuumanschluss 88 angeordnet sein, der mit dem Durchgang durch den Austragskolben 53 verbunden ist. Durch den Vakuumanschluss 88 kann Gas aus dem Innenraum 52 der Kartusche 51 evakuiert werden.

Zur Führung des Mischstabs 57 kann der Austragskolben 53 in einem zentralen Bereich als eine Hülse 90 ausgeformt sein. An seiner Verbindung zur Innenwand der Kartusche 51 und/oder zum Mischstab 57 kann der Austragskolben 53 Abstreiflippen 92 aufweisen, mit denen verhindert werden kann, dass der Knochenzement zwischen dem Austragskolben 53 und der Innenwand der Kartusche 51 und zwischen dem Austragskolben 53 und dem Mischstab 57 vordringen und austreten kann.

Bei einer Klemmung des Austragskolbens 53 mit dem Klemmelement 58 kann das Klemmelement 58 auf eine zylindrische äußere Mantelfläche 94 des Austragskolbens 53 drücken. Das umlaufende Klemmelement 58 kann dabei den Austragskolben 53 radial komprimieren. Der Austragskolben 53 kann dabei durch das Klemmelement 58 elastisch verformt werden, was die Klemmung und damit ein Arretieren des Austragskolbens bewirkt. Beim Lösen der Klemmung mit der Auspressvorrichtung kann die Klemmung gelöst werden und die elastische Deformation des Austragskolbens 53 kann wieder rückgängig gemacht werden, indem sich der Austragskolben 53 beziehungsweise die elastisch deformierten Bereiche des Austragskolbens 53, nämlich die Mantelfläche 94, wieder entspannen.

Um ein Herausfallen des Porenfilters 60 zu verhindern, kann der Austragskolben 53 eine Einsteckhülse 98 aufweisen, mit der der Porenfilter 60 gesichert ist und durch den der Mischstab 57 geführt ist.

Der zweite beispielhafte Knochenzementapplikator kann erfindungsgemäß wie folgt angewendet werden. Ein Zementpulver kann in dem Innenraum 52 der Kartusche 51 enthalten sein oder es wird in den Innenraum 52 eingefüllt. Die Austragsöffnung ist vorzugsweise mit einem Verschluss verschlossen. Die Monomerflüssigkeit kann anschließend zugeführt werden. Der Austragskolben 53 kann soweit in die Kartusche 51 eingeschoben werden, dass das Klemmelement 58 den Austragskolben 53 an der Mantelfläche 94 elastisch deformiert und so den Austragskolben 53 einklemmt. Über den Vakuumanschluss 88 kann im Innenraum 52 der Kartusche 51 ein Vakuum erzeugt werden. Das Zementpulver kann mit der Monomerflüssigkeit durch Bewegen des Mischorgans im Innenraum 52 gemischt werden. Dabei bewegt sich der Austragskolben 53 nicht mit dem Mischstab 57, da er durch die Klemmung mit dem Klemmelement 58 arretiert ist. Das Mischorgan kann mit dem Mischstab 57 an den arretierten Austragskolben 53 gezogen werden und der Mischstab 57 kann abgebrochen werden.

Anschließend kann der Knochenzementapplikator in eine Auspressvorrichtung eingesetzt werden. Beim Antreiben des Austragskolbens 53 kann zunächst die Klemmung gelöst werden und so der Austragskolben 53 von dem Klemmelement 58 gelöst werden. Der Verschluss kann aus der Austragsöffnung entfernt werden und es kann ein Austragsrohr an der Kartusche 51 befestigt werden. Der Knochenzement kann durch weiteres Vortreiben des Austragskolbens 53 im Innenraum 52 der Kartusche 51 aus der Austragsöffnung ausgedrückt werden. Dort kann der Knochenzementteig durch das Austragsrohr ausgepresst werden.

In den Figuren 11 und 12 ist ein Ausschnitt eines dritten erfindungsgemäßen Knochenzementapplikators als Querschnittansicht gezeigt. In den Figuren ist die Vorderseite des Knochenzementapplikators unten und die Rückseite des Knochenzementapplikators oben angeordnet. Der dritte erfindungsgemäße Knochenzementapplikator gleicht dem ersten nach den Figuren 1 bis 9, bis auf den einteiligen Aufbau des Austragskolbens 103 und die fehlende Durchführung für den Mischstab.

Gemäß dem dritten Ausführungsbeispiel der vorliegenden Erfindung kann der Knochenzementapplikator eine röhrenförmigen Kartusche 101 aus einem Kunststoff mit einem zylindrischen Innenraum 102 aufweisen. Im zylindrischen Innenraum 102 der Kartusche 101 kann der in axialer Richtung beweglich gelagerter Austragskolben 103 angeordnet sein. An einer Vorderseite der Kartusche (in den Figuren 11 und 12 nicht zu sehen) kann der zylindrische Innenraum 102 durch einen Kartuschenkopf verschlossen sein. In dem Kartuschenkopf kann eine Austragsöffnung angeordnet sein, durch die ein Knochenzement (in Figur 11 und 12 nicht gezeigt), der im zylindrischen Innenraum 102 der Kartusche 101 enthalten sein kann beziehungsweise gemischt werden kann, mit dem Austragskolben 103 aus dem Innenraum 102 ausgetrieben werden kann. Die Austragsöffnung kann verschließbar oder verschlossen sein. Im zylindrischen Innenraum 102 der Kartusche 101 kann ein Zementpulver (nicht gezeigt) als Ausgangskomponente des herzustellenden Knochenzements angeordnet sein. Eine Monomerflüssigkeit (nicht gezeigt) als zweite Ausgangskomponente kann zu dem Zementpulver in den zylindrischen Innenraum 102 gefüllt werden, um den Knochenzement in dem Innenraum zu mischen.

Zum Durchmischen des Inhalts des Innenraums 102 kann ein Mischorgan (in Figur 11 und 12 nicht zu sehen, aber analog dem ersten Ausführungsbeispiel) in dem zylindrischen Innenraum 102 beweglich angeordnet sein. Das Mischorgan kann mit einem Mischstab (in den Figuren 11 und 12 nicht zu sehen) in dem zylindrischen Innenraum 102 axial bewegt und um die Achse des Mischstabs gedreht werden. Der Mischstab kann durch die Austragsöffnung im Kartuschenkopf in den Innenraum 102 der Kartusche 101 geführt sein.

An der Innenwand der Kartusche 101 kann im Bereich des rückseitigen Endes der Kartusche 101 (in den Figuren oben) eine vorspringende Leiste als Klemmelement 108 zum Festklemmen des Austragskolbens 103 angeordnet sein. Wenn der Austragskolben 103 in das rückseitige Ende der Kartusche 101 eingedrückt wird, kann das Klemmelement 108 einen radialen Druck auf den Austragskolben 103 ausüben. Durch den radialen Druck kann der Austragskolben 103 elastisch deformiert werden. Die elastische Deformation des Austragskolbens 103 kann eine Klemmung des Austragskolbens 103 in der Höhe des Klemmelements 108 bewirken. Wie in Figur 12 zu erkennen ist, kann das Klemmelement als nicht durchgehende Leiste mit Unterbrechungen ausgeführt sein, um die Klemmwirkung aufgrund der kleineren Kontaktfläche zu erhöhen.

In einem Durchgang durch den Austragskolben 103 kann ein für Gase durchlässiger aber für das Zementpulver undurchlässiger Porenfilter 110 angeordnet sein. Durch den Durchgang im Austragskolben 103 und gegebenenfalls durch den Porenfilter 110 kann der Innenraum 102 der Kartusche 101 und dessen Inhalt mit Hilfe eines sterilisierenden Gases wie Ethylenoxid sterilisiert werden und der Innenraum 102 der Kartusche 101 evakuiert werden, um den Knochenzement unter Vakuum mischen zu können.

Um eine Evakuierung des Innenraums 102 zu ermöglichen, können an der Außenwandung des Austragskolbens 103 zwei umlaufende Dichtungen 112 aus Gummi angeordnet sein, mit dem der Austragskolben 103 gegen die Innenwand der Kartusche 101 und damit den zylindrischen Innenraum 102 abgedichtet ist.

An der Rückseite der Kartusche 101 können außen mehrere Befestigungsmittel 116 angeordnet sein, an denen einen Auspressvorrichtung (nicht gezeigt aber analog Figur 3) befestigt werden kann.

Mit dem Mischstab kann das Mischorgan im Innenraum 102 der Kartusche 101 bewegt werden kann. Der Mischstab kann in einer Durchführung durch den Kartuschenkopf beweglich gelagert sein. Der Mischstab kann durch die Durchführung im Kartuschenkopf axial bewegt werden und vorzugsweise auch in der Durchführung gedreht werden. Wenn das Mischorgan starr mit dem Mischstab verbunden ist, kann dementsprechend das Mischorgan im Innenraum 102 der Kartusche 101 axial bewegt werden und vorzugsweise auch gedreht werden. Hierdurch ist der Inhalt der Kartusche 101 mit dem Mischorgan manuell mischbar.

Zum Lösen der Klemmung des Austragskolbens 103 mit dem Klemmelement 108 (siehe Figur 11) kann eine Auspressvorrichtung analog Figur 3 verwendet werden. Mit der Auspressvorrichtung 30 kann nicht nur die Klemmung (siehe Figur 11) gelöst werden, sondern auch der Austragskolben 103 weiter in Richtung des Kartuschenkopfs getrieben werden und dabei der Inhalte der Kartusche 101 (also der Knochenzement) durch die Austragsöffnung aus dem Innenraum der Kartusche 101 ausgetrieben werden.

Zur Evakuierung des Innenraums 102 der Kartusche 101 kann an dem Austragskolben 103 ein Vakuumanschluss 138 angeordnet sein, der mit dem Durchgang durch den Austragskolben 103 verbunden ist. Durch den Vakuumanschluss 138 kann Gas aus dem Innenraum 102 der Kartusche 101 evakuiert werden.

An seiner Verbindung zur Innenwand der Kartusche 101 kann der Austragskolben 103 Abstreiflippen 142 aufweisen, mit denen verhindert werden kann, dass der Knochenzement zwischen dem Austragskolben 103 und der Innenwand der Kartusche 101 vordringen und austreten kann.

Bei einer Klemmung des Austragskolbens 103 mit dem Klemmelement 108 kann das Klemmelement 108 auf eine zylindrische äußere Mantelfläche 144 des Austragskolbens 103 drücken. Das umlaufende Klemmelement 108 kann dabei den Austragskolben 103 radial komprimieren. Der Austragskolben 103 kann dabei durch das Klemmelement 108 elastisch verformt werden, was die Klemmung und damit ein Arretieren des Austragskolbens bewirkt. Beim Lösen der Klemmung mit der Auspressvorrichtung kann die Klemmung gelöst werden und die elastische Deformation des Austragskolbens 103 kann wieder rückgängig gemacht werden, indem sich der Austragskolben 103 beziehungsweise die elastisch deformierten Bereiche des Austragskolbens 103, nämlich die Mantelfläche 144, wieder entspannen.

Der dritte beispielhafte Knochenzementapplikator kann erfindungsgemäß wie folgt angewendet werden. Ein Zementpulver kann in dem Innenraum 102 der Kartusche 101 enthalten sein oder es wird in den Innenraum 102 eingefüllt. Die Austragsöffnung ist vorzugsweise mit einem Verschluss verschlossen. Die Monomerflüssigkeit kann anschließend zugeführt werden. Der Austragskolben 103 kann soweit in die Kartusche 101 eingeschoben werden, dass das Klemmelement 108 den Austragskolben 103 an der Mantelfläche 94 elastisch deformiert und so den Austragskolben 103 einklemmt. Über den Vakuumanschluss 138 kann im Innenraum 102 der Kartusche 101 ein Vakuum erzeugt werden. Das Zementpulver kann mit der Monomerflüssigkeit durch Bewegen des Mischorgans im Innenraum 102 gemischt werden. Dabei bewegt sich der Austragskolben 103 nicht, da er durch die Klemmung mit dem Klemmelement 108 arretiert ist. Das Mischorgan kann mit dem Mischstab an den Kartuschenkopf gezogen werden und der Mischstab kann herausgezogen und damit die Austragsöffnung geöffnet werden.

Anschließend kann der Knochenzementapplikator in eine Auspressvorrichtung eingesetzt werden. Beim Antreiben des Austragskolbens 103 kann zunächst die Klemmung gelöst werden und so der Austragskolben 103 von dem Klemmelement 108 gelöst werden. Der Verschluss kann aus der Austragsöffnung entfernt werden und es kann ein Austragsrohr an der Kartusche 101 befestigt werden. Der Knochenzement kann durch weiteres Vortreiben des Austragskolbens 103 im Innenraum 102 der Kartusche 101 aus der Austragsöffnung ausgedrückt werden. Dort kann der Knochenzementteig durch das Austragsrohr ausgepresst werden.

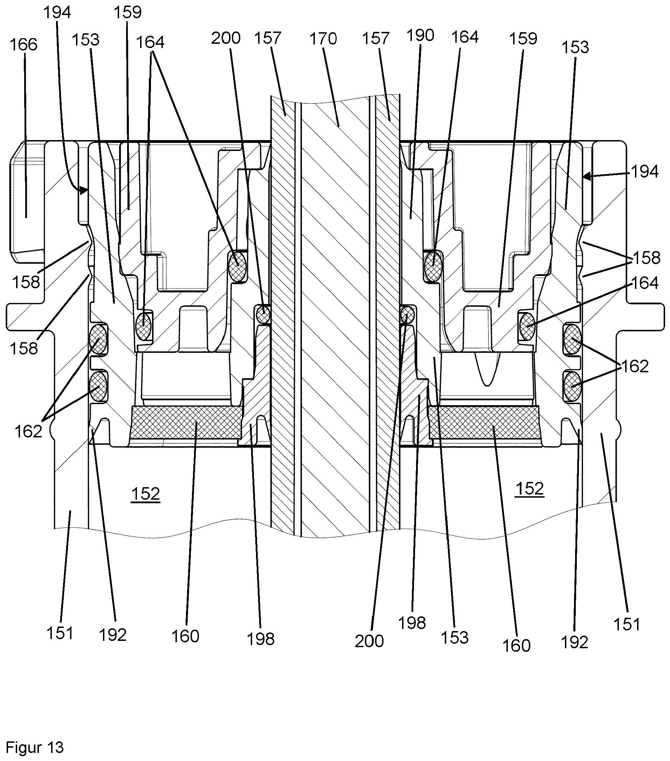

In Figur 13 ist ein Ausschnitt eines vierten erfindungsgemäßen Knochenzementapplikators als Querschnittansicht gezeigt. In der Figur ist die Vorderseite des Knochenzementapplikators unten und die Rückseite des Knochenzementapplikators oben angeordnet. Der vierte erfindungsgemäße Knochenzementapplikator gleicht dem ersten nach den Figuren 1 bis 9, bis auf die doppelt ausgeführten Klemmelemente 158. Im Gegensatz zum ersten Ausführungsbeispiel sind zwei umlaufende vorspringende Leisten als Klemmelemente 158 vorgesehen.

Gemäß dem vierten Ausführungsbeispiel der vorliegenden Erfindung kann der Knochenzementapplikator eine röhrenförmigen Kartusche 151 aus einem Kunststoff mit einem zylindrischen Innenraum 152 aufweisen. Im zylindrischen Innenraum 152 der Kartusche 151 kann der in axialer Richtung beweglich gelagerter Austragskolben 153 angeordnet sein. An einer Vorderseite der Kartusche (in Figur 13 nicht zu sehen) kann der zylindrische Innenraum 152 durch einen Kartuschenkopf verschlossen sein. In dem Kartuschenkopf kann eine Austragsöffnung angeordnet sein, durch die ein Knochenzement (in Figur 13 nicht gezeigt), der im zylindrischen Innenraum 152 der Kartusche 151 enthalten sein kann beziehungsweise gemischt werden kann, mit dem Austragskolben 153 aus dem Innenraum 152 ausgetrieben werden kann. Die Austragsöffnung kann verschließbar oder verschlossen sein. Im zylindrischen Innenraum 152 der Kartusche 151 kann ein Zementpulver (nicht gezeigt) als Ausgangskomponente des herzustellenden Knochenzements angeordnet sein. Eine Monomerflüssigkeit (nicht gezeigt) als zweite Ausgangskomponente kann zu dem Zementpulver in den zylindrischen Innenraum 152 gefüllt werden, um den Knochenzement in dem Innenraum zu mischen.

Zum Durchmischen des Inhalts des Innenraums 152 kann ein Mischorgan (in Figur 13 nicht zu sehen, aber analog dem ersten Ausführungsbeispiel) in dem zylindrischen Innenraum 152 beweglich angeordnet sein. Das Mischorgan kann mit einem Mischstab 157 in dem zylindrischen Innenraum 152 axial bewegt und um die Achse des Mischstabs 157 gedreht werden. Der Mischstab 157 kann durch eine Durchführung im Austragskolben 153 in den Innenraum 152 der Kartusche 151 geführt sein.

An der Innenwand der Kartusche 151 können im Bereich des rückseitigen Endes der Kartusche 151 (in Figur 13 oben) zwei umlaufende vorspringende Leisten als Klemmelemente 158 zum Festklemmen des Austragskolbens 153 angeordnet sein. Wenn der Austragskolben 153 in das rückseitige Ende der Kartusche 151 eingedrückt wird, können die Klemmelemente 158 einen radialen Druck auf den Austragskolben 153 ausüben. Durch den radialen Druck kann der Austragskolben 153 elastisch deformiert werden. Die elastische Deformation des Austragskolbens 153 kann eine Klemmung des Austragskolbens 153 in der Höhe des Klemmelements 158 bewirken.

In der Rückseite des Austragskolbens 153 kann ein Dichtungskolben 159 angeordnet sein, mit dem ein Durchgang durch den Austragskolben 153 abgedichtet werden kann. In dem Durchgang durch den Austragskolben 153 kann ein für Gase durchlässiger aber für das Zementpulver undurchlässiger Porenfilter 160 angeordnet sein. Durch den Durchgang im Austragskolben 153 und gegebenenfalls durch den Porenfilter 160 kann der Innenraum 152 der Kartusche 151 und dessen Inhalt mit Hilfe eines sterilisierenden Gases wie Ethylenoxid sterilisiert werden. Der Dichtungskolben 159 kann anschließend eingeschoben werden, um den Durchgang abzudichten. Bei abgedichtetem Durchgang kann der Innenraum 152 der Kartusche 151 evakuiert werden, um den Knochenzement unter Vakuum mischen zu können.

Um eine Evakuierung des Innenraums 152 zu ermöglichen, können an der Außenwandung des Austragskolbens 153 zwei umlaufende Dichtungen 162 aus Gummi angeordnet sein, mit dem der Austragskolben 153 gegen die Innenwand der Kartusche 151 und damit den zylindrischen Innenraum 152 abgedichtet ist. Ebenso kann der Dichtungskolben 159 gegen den Austragskolben 153 mit einer umlaufenden Dichtung 164 abgedichtet sein. Ferner kann der Austragskolben 153 gegen den Mischstab 157 mit einer umlaufenden Dichtung 200 abgedichtet sein.

An der Rückseite der Kartusche 151 können außen mehrere Befestigungsmittel 166 angeordnet sein, an denen einen Auspressvorrichtung (nicht gezeigt aber analog Figur 3) befestigt werden kann.

Mit dem Mischstab 157 kann das Mischorgan im Innenraum 152 der Kartusche 151 bewegt werden kann. Ein Kern 170 kann im Inneren des Mischstabs 157 angeordnet sein, der den Mischstab 157 mechanisch stabilisiert. Der Mischstab 157 kann in einer Durchführung durch den Austragskolben 153 beweglich gelagert sein. Der Mischstab 157 kann durch die Durchführung durch den Austragskolben 153 axial bewegt werden und vorzugsweise auch in der Durchführung gedreht werden. Wenn das Mischorgan starr mit dem Mischstab 157 verbunden ist, kann dementsprechend das Mischorgan im Innenraum 152 der Kartusche 151 axial bewegt werden und vorzugsweise auch gedreht werden. Hierdurch ist der Inhalt der Kartusche 151 mit dem Mischorgan manuell mischbar.

Zum Lösen der Klemmung des Austragskolbens 153 mit dem Klemmelement 158 kann eine Auspressvorrichtung analog Figur 3 verwendet werden. Mit der Auspressvorrichtung 30 kann nicht nur die Klemmung gelöst werden, sondern auch der Austragskolben 153 weiter in Richtung des Kartuschenkopfs getrieben werden und dabei der Inhalte der Kartusche 151 (also der Knochenzement) durch die Austragsöffnung aus dem Innenraum der Kartusche 151 ausgetrieben werden.

Zur Evakuierung des Innenraums 152 der Kartusche 151 kann an dem Austragskolben 153 ein Vakuumanschluss (nicht gezeigt) angeordnet sein. Durch den Vakuumanschluss kann Gas aus dem Innenraum 152 der Kartusche 151 evakuiert werden.

Zur Führung des Mischstabs 157 kann der Austragskolben 153 in einem zentralen Bereich als eine Hülse 190 ausgeformt sein. An seiner Verbindung zur Innenwand der Kartusche 151 und/oder zum Mischstab 157 kann der Austragskolben 153 Abstreiflippen 192 aufweisen, mit denen verhindert werden kann, dass der Knochenzement zwischen dem Austragskolben 153 und der Innenwand der Kartusche 151 und zwischen dem Austragskolben 153 und dem Mischstab 157 vordringen und austreten kann.

Bei einer Klemmung des Austragskolbens 153 mit dem Klemmelement 158 kann das Klemmelement 158 auf eine zylindrische äußere Mantelfläche 194 des Austragskolbens 153 drücken. Das umlaufende Klemmelement 158 kann dabei den Austragskolben 153 radial komprimieren. Der Austragskolben 153 kann dabei durch das Klemmelement 158 elastisch verformt werden, was die Klemmung und damit ein Arretieren des Austragskolbens bewirkt. Beim Lösen der Klemmung mit der Auspressvorrichtung kann die Klemmung gelöst werden und die elastische Deformation des Austragskolbens 153 kann wieder rückgängig gemacht werden, indem sich der Austragskolben 153 beziehungsweise die elastisch deformierten Bereiche des Austragskolbens 153, nämlich die Mantelfläche 194, wieder entspannen.

Um ein Herausfallen des Porenfilters 160 zu verhindern, kann der Austragskolben 153 eine Einsteckhülse 198 aufweisen, mit der der Porenfilter 160 gesichert ist und durch den der Mischstab 157 geführt ist.

Der vierte beispielhafte Knochenzementapplikator kann erfindungsgemäß wie folgt angewendet werden. Ein Zementpulver kann in dem Innenraum 152 der Kartusche 151 enthalten sein oder es wird in den Innenraum 152 eingefüllt. Die Austragsöffnung ist vorzugsweise mit einem Verschluss verschlossen. Die Monomerflüssigkeit kann anschließend zugeführt werden. Der Austragskolben 153 kann soweit in die Kartusche 151 eingeschoben werden, dass die axial voneinander beabstandeten Klemmelemente 158 den Austragskolben 153 an der Mantelfläche 194 elastisch deformieren und so den Austragskolben 153 einklemmen. Über den Vakuumanschluss kann im Innenraum 152 der Kartusche 151 ein Vakuum erzeugt werden. Das Zementpulver kann mit der Monomerflüssigkeit durch Bewegen des Mischorgans im Innenraum 152 gemischt werden. Dabei bewegt sich der Austragskolben 153 nicht mit dem Mischstab 157, da er durch die Klemmung mit dem Klemmelement 158 arretiert ist. Das Mischorgan kann mit dem Mischstab 157 an den arretierten Austragskolben 153 gezogen werden und der Mischstab 157 kann abgebrochen werden.

Anschließend kann der Knochenzementapplikator in eine Auspressvorrichtung eingesetzt werden. Beim Antreiben des Austragskolbens 153 kann zunächst die Klemmung gelöst werden und so der Austragskolben 153 von den Klemmelementen 158 gelöst werden. Der Verschluss kann aus der Austragsöffnung entfernt werden und es kann ein Austragsrohr an der Kartusche 151 befestigt werden. Der Knochenzement kann durch weiteres Vortreiben des Austragskolbens 153 im Innenraum 152 der Kartusche 151 aus der Austragsöffnung ausgedrückt werden. Dort kann der Knochenzementteig durch das Austragsrohr ausgepresst werden.

### Bezugszeichenliste

- 1, 51, 101, 151: Kartusche
- 2, 52, 102, 152: Innenraum
- 3, 53, 103, 153: Austragskolben
- 4: Kartuschenkopf
- 5: Austragsöffnung
- 6: Mischorgan
- 7, 57, 157: Mischstab
- 8, 58, 108, 158: Klemmelement
- 9, 159: Dichtungskolben
- 10, 60, 110, 160: Porenfilter
- 12, 62, 112, 162: Dichtung
- 14, 64, 164: Dichtung
- 16, 66, 116, 166: Befestigungsmittel
- 18: Griff
- 20, 70, 170: Kern
- 22: Mischflügel
- 24: Stutzen
- 26: Innengewinde
- 28: Stopfen
- 30: Auspressvorrichtung
- 32: Schubstange
- 34: Teller
- 36: Gegenbefestigungsmittel
- 38, 88, 138: Vakuumanschluss
- 40, 90, 190: Hülse
- 42, 92, 142, 192: Abstreiflippe
- 44, 94, 144, 194: Mantelfläche
- 46: Strebe
- 48, 98, 198: Einsteckhülse zur Befestigung des Porenfilters
- 50, 200: Dichtung

## Patentansprüche

1. Knochenzementapplikator zum Bereitstellen eines Knochenzements, insbesondere eines Polymethylmethacrylat-Knochenzements, der Knochenzementapplikator aufweisend
eine Kartusche (1, 51, 101, 151) mit einem zylindrischen Innenraum (2, 52, 102, 152), einen Kartuschenkopf (4) mit einer Austragsöffnung (5) zum Austreiben des Knochenzements, wobei der Kartuschenkopf (4) den zylindrischen Innenraum (2, 52, 102, 152) der Kartusche (1, 51, 101, 151) an einer Vorderseite der Kartusche (1, 51, 101, 151) bis auf die Austragsöffnung (5) verschließt,
ein Mischorgan (6), das im zylindrischen Innenraum (2, 52, 102, 152) der Kartusche (1, 51, 101, 151) beweglich angeordnet ist und im Innenraum (2, 52, 102, 152) mit einem in den Innenraum (2, 52, 102, 152) geführten Mischstab (7, 57, 157) bewegbar ist,
einen Austragskolben (3, 53, 103, 153), der in der Kartusche (1, 51, 101, 151) angeordnet ist und der im zylindrischen Innenraum (2, 52, 102, 152) der Kartusche (1, 51, 101, 151) in Richtung der Austragsöffnung (5) drückbar gelagert ist, wobei der Austragskolben (3, 53, 103, 153) eine äußere zylindrische Mantelfläche (44, 94, 144, 194) aufweist und wobei der Austragskolben (3, 53, 103, 153) mit seiner äußeren zylindrischen Mantelfläche (44, 94, 144, 194) zumindest bereichsweise an einer den zylindrischen Innenraum (2, 52, 102, 152) begrenzenden Innenwand der Kartusche (1, 51, 101, 151) anliegt, **dadurch gekennzeichnet, dass** der Knochenzementapplikator weiterhin aufweist zumindest ein Klemmelement (8, 58, 108, 158), das im Bereich einer der Vorderseite der Kartusche (1, 51, 101, 151) gegenüberliegenden Rückseite des zylindrischen Innenraums (2, 52, 102, 152) an der den zylindrischen Innenraum (2, 52, 102, 152) begrenzenden Innenwand der Kartusche (1, 51, 101, 151) angeordnet ist, wobei das zumindest eine Klemmelement (8, 58, 108, 158) aus der Innenwand der Kartusche (1, 51, 101, 151) in Richtung des zylindrischen Innenraums (2, 52, 102, 152) vorsteht,
wobei
die zylindrische Mantelfläche (44, 94, 144, 194) des Austragskolbens (3, 53, 103, 153) zumindest bereichsweise von dem zumindest einen Klemmelement (8, 58, 108, 158) in Richtung einer zentralen Zylinderachse des zylindrischen Innenraums (2, 52, 102, 152) elastisch deformierbar ist, so dass der Austragskolben (3, 53, 103, 153) mit dem zumindest einen Klemmelement (8, 58, 108, 158) an der Rückseite der Kartusche (1, 51, 101, 151) einklemmbar ist.

2. Knochenzementapplikator nach Anspruch 1, **dadurch gekennzeichnet, dass** das zumindest eine Klemmelement (8, 58, 108, 158) einteilig mit der Kartusche (1, 51, 101, 151) ausgebildet ist.

3. Knochenzementapplikator nach Anspruch 1 oder 2, **dadurch gekennzeichnet, dass** die Austragsöffnung (5) in dem Kartuschenkopf (4) mit einem lösbaren Verschluss verschlossen oder verschließbar ist, wobei bevorzugt ein Gewinde (26) an dem Kartuschenkopf (4) angeordnet ist, in das oder auf das der Verschluss mit einem zu dem Gewinde (26) passenden Gegengewinde geschraubt ist oder schraubbar ist, um die Austragsöffnung (5) zu verschließen.

4. Knochenzementapplikator nach einem der vorangehenden Ansprüche, **dadurch gekennzeichnet, dass**
ein Außendurchmesser des Austragskolbens (3, 53, 103, 153) gleich oder kleiner ist als der Innendurchmesser des zylindrischen Innenraums (2, 52, 102, 152) der Kartusche (1, 51, 101, 151), wobei bevorzugt am Außenumfang des Austragskolbens (3, 53, 103, 153) zumindest eine umlaufende Dichtung (12, 62, 112, 162) und/oder eine umlaufende Abstreiflippe (42, 92, 142, 192) angeordnet ist.

5. Knochenzementapplikator nach einem der vorangehenden Ansprüche, **dadurch gekennzeichnet, dass**
der Innendurchmesser des zylindrischen Innenraums (2, 52, 102, 152) der Kartusche (1, 51, 101, 151) durch das zumindest eine Klemmelement (8, 58, 108, 158) im Bereich des zumindest einen Klemmelements (8, 58, 108, 158) reduziert ist, wobei vorzugsweise der Innendurchmesser so weit reduziert ist, dass der Austragskolben (3, 53, 103, 153) einen größeren Außendurchmesser aufweist als der durch das zumindest eine Klemmelement (8, 58, 108, 158) reduzierte Innendurchmesser.

6. Knochenzementapplikator nach einem der vorangehenden Ansprüche, **dadurch gekennzeichnet, dass**
das zumindest eine Klemmelement (8, 58, 108, 158) ein umlaufender geschlossener Wulst oder Ausschnitte eines umlaufenden Wulsts oder zumindest zwei in axialer Richtung voneinander beabstandete umlaufende geschlossene Wulste oder zumindest zwei in axialer Richtung voneinander beabstandete Ausschnitte eines umlaufenden Wulsts sind.

7. Knochenzementapplikator nach einem der vorangehenden Ansprüche, **dadurch gekennzeichnet, dass**
das zumindest eine Klemmelement (8, 58, 108, 158) eine Fase an einer der Vorderseite der Kartusche (1, 51, 101, 151) zugewandten Vorderseite des zumindest einen Klemmelements (8, 58, 108, 158) und eine Fase an einer der Rückseite der Kartusche (1, 51, 101, 151) zugewandten Rückseite des zumindest einen Klemmelements (8, 58, 108, 158) hat, wobei die Fasen senkrecht zur Zylinderachse der Kartusche (1, 51, 101, 151) angeordnet sind.

8. Knochenzementapplikator nach einem der vorangehenden Ansprüche, **dadurch gekennzeichnet, dass**
zwei oder mehrere Klemmelemente (158) axial bezüglich der Zylinderachse des Innenraums (2, 52, 102, 152) voneinander beabstandet an der Innenwand des zylindrischen Innenraums (2, 52, 102, 152) angeordnet sind.

9. Knochenzementapplikator nach einem der vorangehenden Ansprüche, **dadurch gekennzeichnet, dass**
eine der Innenwand des zylindrischen Innenraums (2, 52, 102, 152) zugewandte Außenwand des Austragskolbens (3, 53, 103, 153) eine axiale Erstreckung besitzt, die zumindest gleich groß der axialen Erstreckung des zumindest einen Klemmelements (8, 58, 108, 158) ist, wobei bevorzugt die axiale Erstreckung der Außenwand des Austragskolbens (3, 53, 103, 153) mindestens zweimal so groß ist, wie die axiale Erstreckung des zumindest einen Klemmelements (8, 58, 108, 158).

10. Knochenzementapplikator nach einem der vorangehenden Ansprüche, **dadurch gekennzeichnet, dass**
eine der Innenwand des zylindrischen Innenraums (2, 52, 102, 152) zugewandte Außenwand des Austragskolbens (3, 53, 103, 153) als geschlossener Hohlzylinder ausgebildet ist, und/oder
der von dem zumindest einen Klemmelement (8, 58, 108, 158) eingeklemmte Austragskolben (3, 53, 103, 153) durch Einwirkung einer Kraft, die längs der Zylinderachse der Kartusche (1, 51, 101, 151) gerichtet ist, lösbar ist und in Richtung der Vorderseite der Kartusche (1, 51, 101, 151) drückbar ist.

11. Knochenzementapplikator nach einem der vorangehenden Ansprüche, **dadurch gekennzeichnet, dass**
die äußere zylindrische Mantelfläche (44, 94, 144, 194) des Austragskolbens (3, 53, 103, 153) aus einem thermoplastischen Kunststoff besteht, wobei bevorzugt der thermoplastische Kunststoff ausgewählt ist aus zumindest einem der Kunststoffe Polyethylen, Polypropylen, Polyamid, Polyethylenterephthalat und Polybutylenterephthalat, und/oder
der Knochenzementapplikator einen Dichtungskolben (9, 159) aufweist, der mit dem Austragskolben (3, 153) verbindbar ist, wobei der Austragskolben (3, 153) die äußere zylindrische Mantelfläche (44, 194) und einen für Gase durchlässigen aber für das Zementpulver undurchlässigen Durchgang umfasst und wobei der Durchgang im Austragskolben (3, 153) mit dem Dichtungskolben (9, 159) dicht verschließbar ist, insbesondere durch Einschieben des Dichtungskolbens (9, 159) in eine in Richtung der Rückseite der Kartusche (1, 151) offene Öffnung des Austragskolbens (3, 153) verschließbar ist, wobei der Durchgang innerhalb der Öffnung des Austragskolbens (3, 153) angeordnet ist.

12. Verfahren zur Herstellung eines Knochenzements, insbesondere eines pastenförmigen Polymethylmethacrylat-Knochenzements, wobei der Knochenzementteig aus einem Zementpulver und einer Monomerflüssigkeit mit einem Knochenzementapplikator nach einem der vorangehenden Ansprüche hergestellt wird, **gekennzeichnet durch** die folgenden nacheinander ablaufenden Schritte:
A) Einfüllen des Zementpulvers und der Monomerflüssigkeit in den zylindrischen Innenraum (2, 52, 102, 152) der Kartusche (1, 51, 101, 151) oder der Monomerflüssigkeit zu dem Zementpulver im zylindrischen Innenraum (2, 52, 102, 152) der Kartusche (1, 51, 101, 151),
B) Verschließen der Kartusche (1, 51, 101, 151) durch Eindrücken des Austragskolbens (3, 53, 103, 153) in den zylindrischen Innenraum (2, 52, 102, 152) der Kartusche (1, 51, 101, 151),
C) Einklemmen des Austragskolbens (3, 53, 103, 153) an der Rückseite der Kartusche (1, 51, 101, 151) mit dem zumindest einen Klemmelement (8, 58, 108, 158),
D) Mischen des Knochenzements im zylindrischen Innenraum (2, 52, 102, 152) der Kartusche (1, 51, 101, 151) durch Bewegen des Mischorgans (6) im zylindrischen Innenraum (2, 52, 102, 152) der Kartusche (1, 51, 101, 151),
E) Eindrücken der Austragskolbens (3, 53, 103, 153) in Richtung der Vorderseite der Kartusche (1, 51, 101, 151), wobei sich dabei die Klemmung des zumindest einen Klemmelements (8, 58, 108, 158) löst, und
F) Auspressen des Knochenzements durch die Austragsöffnung (5) aus der Kartusche (1, 51, 101, 151) durch Vortreiben des Austragskolbens (3, 53, 103, 153) im Innenraum (2, 52, 102, 152) der Kartusche (1, 51, 101, 151) in Richtung des Kartuschenkopfs (4).

13. Verfahren nach Anspruch 12, **dadurch gekennzeichnet, dass**
das Zementpulver vor der Monomerflüssigkeit in den Innenraum (2, 52, 102, 152) der Kartusche (1, 51, 101, 151) gefüllt wird oder bereits in dem zylindrischen Innenraum (2, 52, 102, 152) der Kartusche (1, 51, 101, 151) enthalten ist.

14. Verfahren nach Anspruch 12 oder 13, **dadurch gekennzeichnet, dass**
beim Einklemmen des Austragskolbens (3, 53, 103, 153) in Schritt C) der Austragskolben (3, 53, 103, 153) in Richtung der Zylinderachse des zylindrischen Innenraums (2, 52, 102, 152) der Kartusche (1, 51, 101, 151) elastisch deformiert wird, wobei vorzugsweise ein rohrförmiger Fortsatz an der Rückseite des Austragskolbens (3, 53, 103, 153), dessen Außenfläche zumindest bereichsweise durch die äußere zylindrische Mantelfläche (44, 94, 144, 194) des Austragskolbens (3, 53, 103, 153) begrenzt ist, von dem zumindest einen Klemmelement (8, 58, 108, 158) in Richtung der Zylinderachse des zylindrischen Innenraums (2, 52, 102, 152) der Kartusche (1, 51, 101, 151) gedrückt wird.

15. Verfahren nach einem der Ansprüche 12 bis 14, **dadurch gekennzeichnet, dass**
in Schritt D) das Mischorgan (6) mit dem Mischstab (7, 57, 157) bewegt wird und anschließend mit dem Mischstab (7, 57, 157) gegen eine der Vorderseite der Kartusche (1, 51, 101, 151) zugewandte Vorderseite des Austragskolbens (3, 53, 103, 153) gezogen wird und anschließend der Mischstab (7, 57, 157) abgebrochen wird, und/oder
vor Schritt E) der Knochenzementapplikator in eine Auspressvorrichtung mit einem axial beweglichen und antreibbaren Stößel eingesetzt wird, wobei in Schritt E) und in Schritt F) der Austragskolben (3, 53, 103, 153) mit dem Stößel angetrieben wird, und/oder
die Austragsöffnung (5) verschlossen ist und vor Schritt E) oder F) geöffnet wird.

## Claims

1. A bone cement applicator for providing a bone cement, in particular a polymethyl methacrylate bone cement, the bone cement applicator having
a cartridge (1, 51, 101, 151) with a cylindrical interior (2, 52, 102, 152),
a cartridge head (4) with a delivery opening (5) for discharging the bone cement, wherein the cartridge head (4) closes the cylindrical interior (2, 52, 102, 152) of the cartridge (1, 51, 101, 151) at a front side of the cartridge (1, 51, 101, 151) except for the delivery opening (5),
a mixing member (6) which is movably arranged in the cylindrical interior (2, 52, 102, 152) of the cartridge (1, 51, 101, 151) and is movable in the interior (2, 52, 102, 152) with a mixing rod (7, 57, 157) guided into the interior (2, 52, 102, 152),
a delivery plunger (3, 53, 103, 153) which is arranged in the cartridge (1, 51, 101, 151) and is mounted pressably in the direction of the delivery opening (5) in the cylindrical interior (2, 52, 102, 152) of the cartridge (1, 51, 101, 151), wherein the delivery plunger (3, 53, 103, 153) has an outer cylindrical circumferential surface (44, 94, 144, 194) and wherein the delivery plunger (3, 53, 103, 153) rests at least in places with its outer cylindrical circumferential surface (44, 94, 144, 194) against an inner wall of the cartridge (1, 51, 101, 151), said inner wall defining the cylindrical interior (2, 52, 102, 152), **characterized in that** the bone cement applicator further having
at least one clamping element (8, 58, 108, 158) which is arranged in the region of a back side, opposite the front side of the cartridge (1, 51, 101, 151), of the cylindrical interior (2, 52, 102, 152) on the inner wall of the cartridge (1, 51, 101, 151), said inner wall defining the cylindrical interior (2, 52, 102, 152), wherein the at least one clamping element (8, 58, 108, 158) projects out from the inner wall of the cartridge (1, 51, 101, 151) in the direction of the cylindrical interior (2, 52, 102, 152), wherein
the cylindrical circumferential surface (44, 94, 144, 194) of the delivery plunger (3, 53, 103, 153) is at least in places resiliently deformable by the at least one clamping element (8, 58, 108, 158) in the direction of a central cylinder axis of the cylindrical interior (2, 52, 102, 152), such that the delivery plunger (3, 53, 103, 153) is clampable in place with the at least one clamping element (8, 58, 108, 158) on the back side of the cartridge (1, 51, 101, 151).

2. The bone cement applicator according to Claim 1, **characterized in that**
the at least one clamping element (8, 58, 108, 158) is formed as one part with the cartridge (1, 51, 101, 151).

3. The bone cement applicator according to Claim 1 or 2, **characterized in that**
the delivery opening (5) is closed or closable in the cartridge head (4) with a releasable closure, wherein a thread (26) is preferably arranged on the cartridge head (4), into or onto which the closure is screwed or screwable with a mating thread which fits the thread (26), in order to close the delivery opening (5).

4. The bone cement applicator according to any one of the preceding claims, **characterized in that**
an external diameter of the delivery plunger (3, 53, 103, 153) is the same size as or smaller than the internal diameter of the cylindrical interior (2, 52, 102, 152) of the cartridge (1, 51, 101, 151), wherein at least one circumferential seal (12, 62, 112, 162) and/or a circumferential wiper lip (42, 92, 142, 192) is preferably arranged on the outer circumference of the delivery plunger (3, 53, 103, 153).

5. The bone cement applicator according to any one of the preceding claims, **characterized in that**
the internal diameter of the cylindrical interior (2, 52, 102, 152) of the cartridge (1, 51, 101, 151) is reduced in the region of the at least one clamping element (8, 58, 108, 158) by the at least one clamping element (8, 58, 108, 158), wherein the internal diameter is preferably reduced to such an extent that the delivery plunger (3, 53, 103, 153) has an external diameter larger than the internal diameter reduced by the at least one clamping element (8, 58, 108, 158).

6. The bone cement applicator according to any one of the preceding claims, **characterized in that**
the at least one clamping element (8, 58, 108, 158) is a circumferential, closed bead or portions of a circumferential bead or at least two circumferential, closed beads spaced apart in an axial direction or at least two portions of a circumferential bead spaced apart in an axial direction.

7. The bone cement applicator according to any one of the preceding claims, **characterized in that**
the at least one clamping element (8, 58, 108, 158) has a chamfer on a front side of the at least one clamping element (8, 58, 108, 158), said front side facing towards the front side of the cartridge (1, 51, 101, 151), and a chamfer on a back side of the at least one clamping element (8, 58, 108, 158), said back side facing towards the back side of the cartridge (1, 51, 101, 151), wherein the chamfers are arranged perpendicular to the cylinder axis of the cartridge (1, 51, 101, 151).

8. The bone cement applicator according to any one of the preceding claims, **characterized in that**
two or more clamping elements (158) are arranged, axially spaced apart with regard to the cylinder axis of the interior (2, 52, 102, 152), on the inner wall of the cylindrical interior (2, 52, 102, 152).

9. The bone cement applicator according to any one of the preceding claims, **characterized in that**
an outer wall of the delivery plunger (3, 53, 103, 153), said outer wall facing towards the inner wall of the cylindrical interior (2, 52, 102, 152), has an axial extent which is at least the same size as the axial extent of the at least one clamping element (8, 58, 108, 158), wherein the axial extent of the outer wall of the delivery plunger (3, 53, 103, 153) is preferably at least twice the size of the axial extent of the at least one clamping element (8, 58, 108, 158).

10. The bone cement applicator according to any one of the preceding claims, **characterized in that**
an outer wall of the delivery plunger (3, 53, 103, 153), said outer wall facing towards the inner wall of the cylindrical interior (2, 52, 102, 152), is formed as a closed hollow cylinder, and/or
the delivery plunger (3, 53, 103, 153) clamped in place by the at least one clamping element (8, 58, 108, 158) is releasable by the action of a force directed along the cylinder axis of the cartridge (1, 51, 101, 151) and is pressable in the direction of the front side of the cartridge (1, 51, 101, 151).

11. The bone cement applicator according to any one of the preceding claims, **characterized in that**
the outer cylindrical circumferential surface (44, 94, 144, 194) of the delivery plunger (3, 53, 103, 153) consists of a thermoplastic, wherein the thermoplastic is preferably selected from at least one of the plastics materials polyethylene, polypropylene, polyamide, polyethylene terephthalate and polybutylene terephthalate, and/or
the bone cement applicator has a sealing plunger (9, 159) which is connectable to the delivery plunger (3, 153), wherein the delivery plunger (3, 153) comprises the outer cylindrical circumferential surface (44, 194) and a passage which is permeable to gases but impermeable to the cement powder and wherein the passage in the delivery plunger (3, 153) is impermeably closable by the sealing plunger (9, 159), in particular by inserting the sealing plunger (9, 159) into an opening of the delivery plunger (3, 153), said opening being open in the direction of the back side of the cartridge (1, 151), wherein the passage is arranged within the opening of the delivery plunger (3, 153).

12. A method for producing a bone cement, in particular a paste-like polymethyl methacrylate bone cement, wherein the bone cement paste is produced from a cement powder and a monomer liquid using a bone cement applicator according to any one of the preceding claims, **characterized by** the following succession of steps:
A) introducing the cement powder and the monomer liquid into the cylindrical interior (2, 52, 102, 152) of the cartridge (1, 51, 101, 151) or the monomer liquid to the cement powder in the cylindrical interior (2, 52, 102, 152) of the cartridge (1, 51, 101, 151),
B) closing the cartridge (1, 51, 101, 151) by pressing the delivery plunger (3, 53, 103, 153) into the cylindrical interior (2, 52, 102, 152) of the cartridge (1, 51, 101, 151),
C) clamping the delivery plunger (3, 53, 103, 153) in place on the back side of the cartridge (1, 51, 101, 151) with the at least one clamping element (8, 58, 108, 158),
D) mixing the bone cement in the cylindrical interior (2, 52, 102, 152) of the cartridge (1, 51, 101, 151) by moving the mixing member (6) in the cylindrical interior (2, 52, 102, 152) of the cartridge (1, 51, 101, 151),
E) pressing in the delivery plunger (3, 53, 103, 153) in the direction of the front side of the cartridge (1, 51, 101, 151), wherein the clamping effect of the at least one clamping element (8, 58, 108, 158) is released, and
F) expelling the bone cement from the cartridge (1, 51, 101, 151) through the delivery opening (5) by advancing the delivery plunger (3, 53, 103, 153) in the interior (2, 52, 102, 152) of the cartridge (1, 51, 101, 151) in the direction of the cartridge head (4).

13. The method according to Claim 12, **characterized in that**
the cement powder is introduced into the interior (2, 52, 102, 152) of the cartridge (1, 51, 101, 151) before the monomer liquid or is already present in the cylindrical interior (2, 52, 102, 152) of the cartridge (1, 51, 101, 151).

14. The method according to Claim 12 or 13, **characterized in that**,
on clamping the delivery plunger (3, 53, 103, 153) in place in step C), the delivery plunger (3, 53, 103, 153) is resiliently deformed in the direction of the cylinder axis of the cylindrical interior (2, 52, 102, 152) of the cartridge (1, 51, 101, 151), wherein a tubular extension on the back side of the delivery plunger (3, 53, 103, 153), the outer side of which is defined at least in places by the outer cylindrical circumferential surface (44, 94, 144, 194) of the delivery plunger (3, 53, 103, 153), is preferably pressed by the at least one clamping element (8, 58, 108, 158) in the direction of the cylinder axis of the cylindrical interior (2, 52, 102, 152) of the cartridge (1, 51, 101, 151).

15. The method according to any one of Claims 12 to 14, **characterized in that**,
in step D), the mixing member (6) is moved with the mixing rod (7, 57, 157) and then drawn with the mixing rod (7, 57, 157) against a front side of the delivery plunger (3, 53, 103, 153), which front side faces towards the front side of the cartridge (1, 51, 101, 151), and then the mixing rod (7, 57, 157) is broken off, and/or
before step E), the bone cement applicator is inserted into an expulsion device with an axially mobile and drivable ram, wherein in step E) and in step F) the delivery plunger (3, 53, 103, 153) is advanced with the ram, and/or
the delivery opening (5) is closed and is opened before step E) or F).

## Revendications

1. Applicateur de ciment osseux permettant de fournir un ciment osseux, en particulier un ciment osseux en polyméthacrylate de méthyle, l'applicateur de ciment osseux présentant
une cartouche (1, 51, 101, 151) comportant un espace interne (2, 52, 102, 152) cylindrique, une tête de cartouche (4) comportant une ouverture de décharge (5) pour l'expulsion du ciment osseux, dans lequel la tête de cartouche (4) ferme l'espace interne (2, 52, 102, 152) cylindrique de la cartouche (1, 51, 101, 151) au niveau d'un côté avant de la cartouche (1, 51, 101, 151) jusqu'à l'ouverture de décharge (5),
un organe de mélange (6) qui est disposé mobile dans l'espace interne (2, 52, 102, 152) cylindrique de la cartouche (1, 51, 101, 151) et est mobile dans l'espace interne (2, 52, 102, 152) avec une tige de mélange (7, 57, 157) guidée dans l'espace interne (2, 52, 102, 152),
un piston de décharge (3, 53, 103, 153) qui est disposé dans la cartouche (1, 51, 101, 151) et qui est monté dans l'espace interne (2, 52, 102, 152) cylindrique de la cartouche (1, 51, 101, 151) de manière à pouvoir être pressé en direction de l'ouverture de décharge (5), dans lequel le piston de décharge (3, 53, 103, 153) présente une surface d'enveloppe cylindrique externe (44, 94, 144, 194) et dans lequel le piston de décharge (3, 53, 103, 153) repose avec sa surface d'enveloppe cylindrique externe (44, 94, 144, 194), au moins dans certaines zones, sur une paroi interne de la cartouche (1, 51, 101, 151) délimitant l'espace interne (2, 52, 102, 152) cylindrique,
**caractérisé en ce que** l'applicateur de ciment osseux présente en outre au moins un élément de serrage (8, 58, 108, 158) qui est disposé dans la zone d'un côté arrière de l'espace interne (2, 52, 102, 152) cylindrique opposé au côté avant de la cartouche (1, 51, 101, 151) sur la paroi interne de la cartouche (1, 51, 101, 151) délimitant l'espace interne (2, 52, 102, 152) cylindrique, dans lequel l'au moins un élément de serrage (8, 58, 108, 158) fait saillie depuis la paroi interne de la cartouche (1, 51, 101, 151) en direction de l'espace interne (2, 52, 102, 152) cylindrique, dans lequel
la surface d'enveloppe cylindrique (44, 94, 144, 194) du piston de décharge (3, 53, 103, 153) est déformable élastiquement, au moins dans certaines zones, par l'au moins un élément de serrage (8, 58, 108, 158) en direction d'un axe de cylindre central de l'espace interne (2, 52, 102, 152) cylindrique, de sorte que le piston de décharge (3, 53, 103, 153) peut être serré par l'au moins un élément de serrage (8, 58, 108, 158) sur le côté arrière de la cartouche (1, 51, 101, 151).

2. Applicateur de ciment osseux selon la revendication 1,
**caractérisé en ce que**
l'au moins un élément de serrage (8, 58, 108, 158) est réalisé d'une seule pièce avec la cartouche (1, 51, 101, 151).

3. Applicateur de ciment osseux selon la revendication 1 ou 2,
**caractérisé en ce que**
l'ouverture de décharge (5) dans la tête de cartouche (4) est fermée ou peut être fermée avec une fermeture détachable, dans lequel de préférence un filetage (26) est disposé sur la tête de cartouche (4), dans lequel filetage ou sur lequel filetage la fermeture est vissée ou peut être vissée avec un contre-filetage adapté au filetage (26) afin de fermer l'ouverture de décharge (5).

4. Applicateur de ciment osseux selon l'une des revendications précédentes, **caractérisé en ce que**
un diamètre externe du piston de décharge (3, 53, 103, 153) est égal ou inférieur au diamètre interne de l'espace interne (2, 52, 102, 152) cylindrique de la cartouche (1, 51, 101, 151), dans lequel de préférence, sur la périphérie externe du piston de décharge (3, 53, 103, 153), au moins un joint d'étanchéité périphérique (12, 62, 112, 162) et/ou une lèvre de raclage périphérique (42, 92, 142, 192) sont disposés.

5. Applicateur de ciment osseux selon l'une des revendications précédentes, **caractérisé en ce que**
le diamètre interne de l'espace interne (2, 52, 102, 152) cylindrique de la cartouche (1, 51, 101, 151) est réduit par l'au moins un élément de serrage (8, 58, 108, 158) dans la zone de l'au moins un élément de serrage (8, 58, 108, 158), dans lequel de préférence le diamètre interne est réduit de telle manière que le piston de décharge (3, 53, 103, 153) présente un diamètre externe supérieur au diamètre interne réduit par l'au moins un élément de serrage (8, 58, 108, 158).

6. Applicateur de ciment osseux selon l'une des revendications précédentes, **caractérisé en ce que**
l'au moins un élément de serrage (8, 58, 108, 158) sont un bourrelet périphérique fermé ou des découpes d'un bourrelet périphérique ou au moins deux bourrelets périphériques fermés espacés l'un de l'autre dans la direction axiale ou au moins deux découpes d'un bourrelet périphérique espacées l'une de l'autre dans la direction axiale.

7. Applicateur de ciment osseux selon l'une des revendications précédentes, **caractérisé en ce que**
l'au moins un élément de serrage (8, 58, 108,158) a un chanfrein sur un côté avant de l'au moins un élément de serrage (8, 58, 108, 158) tourné vers le côté avant de la cartouche (1, 51, 101, 151) et un chanfrein sur un côté arrière de l'au moins un élément de serrage (8, 58, 108, 158) tourné vers le côté arrière de la cartouche (1, 51, 101, 151), dans lequel les chanfreins sont disposés perpendiculairement à l'axe de cylindre de la cartouche (1, 51, 101, 151).

8. Applicateur de ciment osseux selon l'une des revendications précédentes, **caractérisé en ce que**
deux éléments de serrage (158) ou plus sont disposés sur la paroi interne de l'espace interne (2, 52, 102, 152) cylindrique de manière à être espacés l'un de l'autre axialement par rapport à l'axe de cylindre de l'espace interne (2, 52, 102, 152).

9. Applicateur de ciment osseux selon l'une des revendications précédentes, **caractérisé en ce que**
une paroi externe du piston de décharge (3, 53, 103, 153) tournée vers la paroi interne de l'espace interne (2, 52, 102, 152) cylindrique possède une extension axiale qui est au moins aussi grande que l'extension axiale de l'au moins un élément de serrage (8, 58, 108, 158), dans lequel de préférence l'extension axiale de la paroi externe du piston de décharge (3, 53, 103, 153) est au moins deux fois plus grande que l'extension axiale de l'au moins un élément de serrage (8, 58, 108, 158).

10. Applicateur de ciment osseux selon l'une des revendications précédentes, **caractérisé en ce que**
une paroi externe du piston de décharge (3, 53, 103, 153) tournée vers la paroi interne de l'espace interne (2, 52, 102, 152) cylindrique est réalisée sous forme de cylindre creux fermé, et/ou
le piston de décharge (3, 53, 103, 153) serré par l'au moins un élément de serrage (8, 58, 108, 158) peut être libéré par l'action d'une force qui est dirigée le long de l'axe de cylindre de la cartouche (1, 51, 101, 151) et peut être pressé en direction du côté avant de la cartouche (1, 51, 101, 151).

11. Applicateur de ciment osseux selon l'une des revendications précédentes, **caractérisé en ce que**
la surface d'enveloppe cylindrique externe (44, 94, 144, 194) du piston de décharge (3, 53, 103, 153) est constituée d'une matière synthétique thermoplastique, dans lequel de préférence la matière synthétique thermoplastique est choisie parmi au moins l'une des matières synthétiques polyéthylène, polypropylène, polyamide, polyéthylène téréphtalate et polybutylène téréphtalate, et/ou
l'applicateur de ciment osseux présente un piston d'étanchéité (9, 159) qui peut être relié au piston de décharge (3, 153), dans lequel le piston de décharge (3, 153) comprend la surface d'enveloppe cylindrique externe (44, 194) et un passage perméable aux gaz mais imperméable à la poudre de ciment et dans lequel le passage peut être fermé de manière étanche dans le piston de décharge (3, 153) par le piston d'étanchéité (9, 159), en particulier peut être fermé par insertion du piston d'étanchéité (9, 159) dans une ouverture du piston de décharge (3, 153) ouverte en direction du côté arrière de la cartouche (1, 151), dans lequel le passage est disposé à l'intérieur de l'ouverture du piston de décharge (3, 153).

12. Procédé de fabrication d'un ciment osseux, en particulier d'un ciment osseux en polyméthacrylate de méthyle pâteux, dans lequel la pâte de ciment osseux est fabriquée à partir d'une poudre de ciment et d'un liquide monomère avec un applicateur de ciment osseux selon l'une des revendications précédentes, **caractérisé par** les étapes suivantes se déroulant successivement :
A) introduction de la poudre de ciment et du liquide monomère dans l'espace interne (2, 52, 102, 152) cylindrique de la cartouche (1, 51, 101, 151) ou du liquide monomère à la poudre de ciment dans l'espace interne (2, 52, 102, 152) cylindrique de la cartouche (1, 51, 101, 151),
B) fermeture de la cartouche (1, 51, 101, 151) par enfoncement du piston de décharge (3, 53, 103, 153) dans l'espace interne (2, 52, 102, 152) cylindrique de la cartouche (1, 51, 101, 151),
C) serrage du piston de décharge (3, 53, 103, 153) sur le côté arrière de la cartouche (1, 51, 101, 151) par l'au moins un élément de serrage (8, 58, 108, 158),
D) mélange du ciment osseux dans l'espace interne (2, 52, 102, 152) cylindrique de la cartouche (1, 51, 101, 151) par déplacement de l'organe de mélange (6) dans l'espace interne (2, 52, 102, 152) cylindrique de la cartouche (1, 51, 101, 151),
E) enfoncement du piston de décharge (3, 53, 103, 153) en direction du côté avant de la cartouche (1, 51, 101, 151), dans lequel le serrage de l'au moins un élément de serrage (8, 58, 108, 158) se libère ainsi, et
F) expulsion par pression du ciment osseux à travers l'ouverture de décharge (5) hors de la cartouche (1, 51, 101, 151) en faisant avancer le piston de décharge (3, 53, 103, 153) dans l'espace interne (2, 52, 102, 152) de la cartouche (1, 51, 101, 151) en direction de la tête de cartouche (4).

13. Procédé selon la revendication 12, **caractérisé en ce que**
la poudre de ciment est introduite avant le liquide monomère dans l'espace interne (2, 52, 102, 152) de la cartouche (1, 51, 101, 151) ou est déjà contenue dans l'espace interne (2, 52, 102, 152) cylindrique de la cartouche (1, 51, 101, 151),

14. Procédé selon la revendication 12 ou 13, **caractérisé en ce que**
lors du serrage du piston de décharge (3, 53, 103, 153) à l'étape C), le piston de décharge (3, 53, 103, 153) est déformé élastiquement en direction de l'axe de cylindre de l'espace interne (2, 52, 102, 152) cylindrique de la cartouche (1, 51, 101, 151), dans lequel de préférence un prolongement tubulaire sur le côté arrière du piston de décharge (3, 53, 103, 153), dont la surface externe est délimitée au moins dans certaines zones par la surface d'enveloppe cylindrique externe (44, 94, 144, 194) du piston de décharge (3, 53, 103, 153), est pressé par l'au moins un élément de serrage (8, 58, 108, 158) en direction de l'axe de cylindre de l'espace interne (2, 52, 102, 152) cylindrique de la cartouche (1, 51, 101, 151).

15. Procédé selon l'une des revendications 12 à 14, **caractérisé en ce que**
à l'étape D), l'organe de mélange (6) est déplacé avec la tige de mélange (7, 57, 157) et est ensuite tiré avec la tige de mélange (7, 57, 157) contre un côté avant du piston de décharge (3, 53, 103, 153) tourné vers le côté avant de la cartouche (1, 51, 101, 151) et ensuite la tige de mélange (7, 57, 157) est rompue, et/ou
avant l'étape E), l'applicateur de ciment osseux est placé dans un dispositif d'expulsion par pression comportant un poussoir mobile axialement et pouvant être entraîné, dans lequel, à l'étape E) et à l'étape F), le piston de décharge (3, 53, 103, 153) est entraîné par le poussoir, et/ou
l'ouverture de décharge (5) est fermée et est ouverte avant l'étape E) ou F).
